# EUROPÄISCHE PATENTSCHRIFT

(11) **EP 1 255 816 B1**
(45) Veröffentlichungstag und Bekanntmachung des Hinweises auf die Patenterteilung: **21.06.2006**
(21) Anmeldenummer: 00972794.2
(22) Anmeldetag: 18.10.2000
(51) Int. Cl.: C12N 9/00, C12N 15/10, C12N 15/62, C12P 7/00

(54) **MASSGESCHNEIDERTE PEPTIDSYNTHETASEN, VERFAHREN ZU IHRER HERSTELLUNG UND IHRE VERWENDUNG**
TAILORED PEPTIDE SYNTHETASES, A METHOD FOR THEIR PRODUCTION AND THE USE THEREOF
SYNTHETASES DE PEPTIDES INDIVIDUALISEES, LEUR PROCEDE DE PRODUCTION ET LEUR UTILISATION

(30) Priorität: 22.10.1999 DE 19951196
(43) Veröffentlichungstag der Anmeldung: 13.11.2002
(73) Patentinhaber: Cubist Pharmaceutical Inc., Lexington, MA 02421 (US)
(72) Erfinder: MARAHIEL, Mohamed A., 35043 Marburg (DE); MOOTZ, Henning, 35039 Marburg (DE); SCHWARZER, Dirk, Baltimore, MD 21201 (US); DÖKEL, Sascha, Cambridge, MA 02140 (US); LINNE, Uwe, 35119 Rosenthal (DE)
(74) Vertreter: Ackermann, Joachim
(86) Internationale Anmeldenummer: PCT/EP2000/010250
(87) Internationale Veröffentlichungsnummer: WO 2001/030985

(56) Entgegenhaltungen:
- WO-A-00/52152
- STACHELHAUS TORSTEN ET AL: "Rational design of peptide antibiotics by targeted replacement of bacterial and fungal domains." SCIENCE (WASHINGTON D C), Bd. 269, Nr. 5220, 1995, Seiten 69-72, XP000941883 ISSN: 0036-8075
- SCHNEIDER A ET AL: "Targeted alteration of the substrate specificity of peptide synthetases by rational module swapping." MOLECULAR & GENERAL GENETICS, Bd. 257, Nr. 3, Februar 1998 (1998-02), Seiten 308-318, XP002141778 ISSN: 0026-8925 in der Anmeldung erwähnt
- MOOTZ H D ET AL: "DESIGN AND APPLICATION OF MULTIMODULAR PEPTIDE SYNTHETASES" CURRENT OPINION IN BIOTECHNOLOGY,LONDON,GB, Bd. 10, Nr. 4, August 1999 (1999-08), Seiten 341-348, XP000892842 ISSN: 0958-1669
- RANGANATHAN ANAND ET AL: "Knowledge-based design of bimodular and trimodular polyketide synthases based on domain and module swaps: A route to simple statin analogues." CHEMISTRY & BIOLOGY (LONDON), Bd. 6, Nr. 10, Oktober 1999 (1999-10), Seiten S1-S4, XP002160079 ISSN: 1074-5521 & corresponding PubMed abstract (PMID:10508677)
- GOKHALE RAJESH S ET AL: "Dissecting and exploiting intermodular communication in polyketide synthases." SCIENCE (WASHINGTON D C), Bd. 284, Nr. 5413, 16. April 1999 (1999-04-16), Seiten 482-485, XP002155932 ISSN: 0036-8075
- DIECKMANN RALF ET AL: "Probing the domain structure and ligand-induced conformational changes by limited proteolysis of tyrocidine synthetase 1." JOURNAL OF MOLECULAR BIOLOGY, Bd. 288, Nr. 1, 23. April 1999 (1999-04-23), Seiten 129-140, XP002160080 ISSN: 0022-2836
- MARAHIEL M A ET AL: "Modular petide synthetases involved in nonribosomal peptide synthesis" CHEMICAL REVIEWS,US,AMERICAN CHEMICAL SOCIETY. EASTON, Bd. 97, Nr. 7, November 1997 (1997-11), Seiten 2651-2673, XP002133489 ISSN: 0009-2665 in der Anmeldung erwähnt
- MOOTZ HENNING D ET AL: "Construction of hybrid peptide synthetases by module and domain fusions." PROCEEDINGS OF THE NATIONAL ACADEMY OF SCIENCES OF THE UNITED STATES, Bd. 97, Nr. 11, 23. Mai 2000 (2000-05-23), Seiten 5848-5853, XP002160081 May 23, 2000 ISSN: 0027-8424

### SEQUENZPROTOKOLL

<110> Marahiel Prof., Mohamed
   Mootz, Henning
   Schwarzer, Dirk
   Dökel, Sascha
   Linne, Uwe
<120> Maßgeschneiderte Peptidsynthetasen, Verfahren zu ihrer Herstellung und ihre Verwendung
<130> 199ma02.wo
<140>
   <141>
<150> 199 51 196.9-41
   <151> 1999-10-22
<160> 60
<170> Patent In Ver. 2.1
<210> 1
   <211> 27
   <212> DNA
   <213> Bacillus brevis
<400> 1
   ataccatggt agcaaatcag gccaatc 27
<210> 2
   <211> 27
   <212> DNA
   <213> Bacillus brevis
<400> 2
   tatggatccg cgcagtctat ttgcaag 27
<210> 3
   <211> 33
   <212> DNA
   <213> Bacillus brevis
<400> 3
   tatccatggg tgtatttagc aaagaacaag ttc 33
<210> 4
   <211> 27
   <212> DNA
   <213> Bacillus brevis
<400> 4
   tatggatcct tccacatacg ctgccag 27
<210> 5
   <211> 30
   <212> DNA
   <213> Bacillus brevis
<400> 5
   ataggatccg ccaaagggaa tgtcttctcg 30
<210> 6
   <211> 30
   <212> DNA
   <213> Bacillus brevis
<400> 6
   ataggatcct ttcaggatga acagttcttg 30
<210> 7
   <211> 27
   <212> DNA
   <213> Bacillus brevis
<400> 7
   ataggatccg cattcgagca gttcgag 27
<210> 8
   <211> 27
   <212> DNA
   <213> Bacillus brevis
<400> 8
   ataggatcct tcgatgaacg ccgccag 27
<210> 9
   <211> 29
   <212> DNA
   <213> Bacillus brevis
<400> 9
   ataagatctc ataagcgctt tgagagcag 29
<210> 10
   <211> 40
   <212> DNA
   <213> Bacillus subtilis
<400> 10
   atagctagcg gaggatcaca tggaaataac tttttaccct 40
<210> 11
   <211> 27
   <212> DNA
   <213> Bacillus subtilis
<400> 11
   atatctagaa tcagcttcct ctgcaag 27
<210> 12
   <211> 28
   <212> DNA
   <213> Bacillus subtilis
<400> 12
   atagctagcg agaaagagaa gctgcttg 28
<210> 13
   <211> 27
   <212> DNA
   <213> Bacillus brevis
<400> 13
   atagctagcg cattcgagca gttcgag 27
<210> 14
   <211> 27
   <212> DNA
   <213> Bacillus brevis
<400> 14
   atatctagat ctcagcatgg tgacatc 27
<210> 15
   <211> 26
   <212> DNA
   <213> Amycolatopsis orientalis
<400> 15
   atagctagcc gcgaaccgcg caccga 26
<210> 16
   <211> 27
   <212> DNA
   <213> Amycolatopsis orientalis
<400> 16
   atatctagat ttgaccatgg ccgccag 27
<210> 17
   <211> 30
   <212> DNA
   <213> Bacillus subtilis
<400> 17
   atagctagca aagaacacgc ttttacacag 30
<210> 18
   <211> 29
   <212> DNA
   <213> Bacillus subtilis
<400> 18
   atatctagat cctctcctat agtttgttg 29
<210> 19
   <211> 27
   <212> DNA
   <213> Bacillus subtilis
<400> 19
   atagctagcg gagattcact gatgccg 27
<210> 20
   <211> 27
   <212> DNA
   <213> Bacillus subtilis
<400> 20
   atatctagaa cggataacgg tagccag 27
<210> 21
   <211> 28
   <212> DNA
   <213> Bacillus brevis
<400> 21
   atagctagcg gaaaagagac gtatgtgc 28
<210> 22
   <211> 26
   <212> DNA
   <213> Bacillus brevis
<400> 22
   atatctagat gtcgtccgct cgccgg 26
<210> 23
   <211> 26
   <212> DNA
   <213> Bacillus brevis
<400> 23
   atagctagcg ctgcctacca tcctcc 26
<210> 24
   <211> 28
   <212> DNA
   <213> Bacillus brevis
<400> 24
   atatctagac aacatctgtt tagcgcag 28
<210> 25
   <211> 26
   <212> DNA
   <213> Bacillus brevis
<400> 25
   atagctagcg gaggtgccgt aatgag 26
<210> 26
   <211> 27
   <212> DNA
   <213> Bacillus brevis
<400> 26
   atatctagat tccacatacg ctgccag 27
<210> 27
   <211> 30
   <212> DNA
   <213> Bacillus licheniformis
<400> 27
   atagctagcc gaaggaaatg cttttacatc 30
<210> 28
   <211> 28
   <212> DNA
   <213> Bacillus licheniformis
<400> 28
   atatctagag acatttacat tgccgtcg 28
<210> 29
   <211> 26
   <212> DNA
   <213> Bacillus brevis
<400> 29
   atagctagcg tgtatgtggc gccgcg 26
<210> 30
   <211> 27
   <212> DNA
   <213> Bacillus brevis
<400> 30
   atatctagag gttacgggct tgccttc 27
<210> 31
   <211> 26
   <212> DNA
   <213> Bacillus licheniformis
<400> 31
   atagctagcg gaaccgggta cgatcc 26
<210> 32
   <211> 29
   <212> DNA
   <213> Bacillus licheniformis
<400> 32
   atatctagat aatacaaaac gcgctaatg 29
<210> 33
   <211> 28
   <212> DNA
   <213> Bacillus subtilis
<400> 33
   atagctagca aacagctgac agcagcaa 28
<210> 34
   <211> 28
   <212> DNA
   <213> Bacillus subtilis
<400> 34
   atatctagat ccgctttatc gtttgtgc 28
<210> 35
   <211> 25
   <212> DNA
   <213> Bacillus licheniformis
<400> 35
   tttccatggc taaacattca ttaga 25
<210> 36
   <211> 25
   <212> DNA
   <213> Bacillus licheniformis
<400> 36
   ttcctgcagc gcccccgccg ttctg 25
<210> 37
   <211> 26
   <212> DNA
   <213> Bacillus brevis
<400> 37
   agcctgcagg cctaccatcc tccgag 26
<210> 38
   <211> 24
   <212> DNA
   <213> Bacillus brevis
<400> 38
   tggacccatg gtaatttctc ctct 24
<210> 39
   <211> 25
   <212> DNA
   <213> Bacillus brevis
<400> 39
   aractgcagg agtatgtagc gccgc 25
<210> 40
   <211> 30
   <212> DNA
   <213> Bacillus brevis
<400> 40
   tatggatcct ttcaggatga acagttcttg 30
<210> 41
   <211> 26
   <212> DNA
   <213> Bacillus brevis
<400> 41
   accgttaacg aatacgtggc cccgag 26
<210> 42
   <211> 24
   <212> DNA
   <213> Bacillus brevis und licheniformis
<400> 42
   aatgttaacc tcctgcagcg cccc 24
<210> 43
   <211> 25
   <212> DNA
   <213> Bacillus brevis
<400> 43
   acgctgcagg attacgtcgc cccga 25
<210> 44
   <211> 27
   <212> DNA
   <213> Bacillus brevis
<400> 44
   agcgttaact gttgcaggct ttccttc 27
<210> 45
   <211> 35
   <212> DNA
   <213> Saccharopolyspora erythraea
<400> 45
   tatccatggg aagatctctc gtcggcgcag cagag 35
<210> 46
   <211> 26
   <212> DNA
   <213> Saccharopolyspora erythraea
<400> 46
   ataggatcct gaattccctc cgccca 26
<210> 47
   <211> 24
   <212> DNA
   <213> Bacillus brevis
<400> 47
   taaagatctg cctaccatcc tccg 24
<210> 48
   <211> 27
   <212> DNA
   <213> Bacillus brevis
<400> 48
   tatggatccg cgcagtgtat ttgcaag 27
<210> 49
   <211> 30
   <212> DNA
   <213> Bacillus brevis
<400> 49
   ataagatcta gaaaaagcga tcagggcatc 30
<210> 50
   <211> 23
   <212> DNA
   <213> Bacillus brevis
<400> 50
   aatgcatgct gactgcgcat gag 23
<210> 51
   <211> 24
   <212> DNA
   <213> Bacillus brevis
<400> 51
   taaggatcct gttgcaggct ttcc 24
<210> 52
   <211> 30
   <212> DNA
   <213> Bacillus brevis
<400> 52
   ataggatcct tcgatcaagc gggccaagtc 30
<210> 53
   <211> 21
   <212> DNA
   <213> Bacillus brevis
<400> 53
   aaagcatgct gacagcagca g 21
<210> 54
   <211> 27
   <212> DNA
   <213> Bacillus brevis
<400> 54
   aaaggatccc cggttctcct cctggtt 27
<210> 55
   <211> 26
   <212> DNA
   <213> Bacillus brevis
<400> 55
   aaaggatccc gggatgacgc gcagag 26
<210> 56
   <211> 25
   <212> DNA
   <213> Bacillus brevis
<400> 56
   aatccatggt cagcgaggaa gagcg 25
<210> 57
   <211> 22
   <212> DNA
   <213> Bacillus brevis
<400> 57
   aaaggatcct gtcgtccgct cg 22
<210> 58
   <211> 27
   <212> DNA
   <213> Bacillus licheniformis
<400> 58
   aaaccatggt tgctaaacat tcattag 27
<210> 59
   <211> 24
   <212> DNA
   <213> Bacillus licheniformis
<400> 59
   aaaggatccc gccccgccgt tctg 24
<210> 60
   <211> 25
   <212> DNA
   <213> Bacillus licheniformis
<400> 60
   aaaggatcct tcgataaaag cgctc 25

## Beschreibung

Die vorliegende Erfindung betrifft neue, maßgeschneiderte nicht-ribosomale Peptidsynthetasen (NRPS), deren Herstellung und die Verwendung dieser Synthetasen zur Synthese bekannter oder auch artifizieller, konstruierter Peptide oder zur Modifikation einzelner Aminosäuren.

Nicht-ribosomale Peptidsynthetasen (NRPS oder Peptidsynthetasen) sind modular aufgebaute Enzyme mit ungewöhnlichen Strukturen und wichtigen biologischen Funktionen. Zahlreiche Peptide mit pharmazeutischem und/ oder biotechnologischem Interesse werden von großen Enzymkomplexen, den sogenannten NRPS (Marahiel et al. (1997), Chem. Rev. 97: S.2651-2673) synthetisiert. Dazu gehören bekannte Medikamente wie Cyclosporin A und Vancomycin. Die große Vielfalt der so synthetisierten bioaktiven Peptide sind ein Ergebnis der großen strukturellen Vielfalt der NRPS. NRPS bauen oft ungewöhnliche Bausteine, wie z.B. α-hydroxy Aminosäuren oder nichtproteinogene Aminosäuren, ein. Die Reste können weiter modifiziert werden, beispielsweise durch N-Methylierung, heterozyklische Ringbildung oder Epimerisierung. Viele der durch natürliche NRPS synthetisierten Peptide sind durch Ester- oder Peptidbindungen zyklisiert. Generell kann man sagen, daß NRPS eine Schlüsselrolle bei der Synthese von komplexen Bioverbindungen spielen.

Es wurde bereits gefunden, daß die multifunktionellen Proteintemplates einen im wesentlichen modularen Aufbau besitzen. Man bezeichnet als Modul die katalytische Einheit, die einen spezifischen Grundbaustein zur Elongation, also meist eine α-Aminosäure, in das Produkt (Peptid) einbaut (Marahiel et al. (1997), Chem. Rev. 97: S.2651-2673). Die Reihenfolge der Module innerhalb der NRPS bestimmt die Abfolge der Bausteine innerhalb des Produkts. Die einzelnen Module setzen sich aus sogenannten Domänen zusammen, die jeweils für einen bestimmten Reaktionsschritt zuständig sind. So bestimmt beispielsweise die Adenylierungsdomäne (A-Domäne) den Eintritt des Substrates in die nicht-ribosomale Peptidsynthese, indem die A-Domäne das Substrat, gewöhnlich eine Aminosäure, selektiert und adenyliert. Die aktivierte Aminosäure kann anschließend über eine Thioesterbindung an den Kofaktor 4'-Phosphopantethein einer Thiolierungsdomäne (T-Domäne) gebunden werden. Die T-Domäne wird in der Fachwelt auch als PCP-Domäne (Peptidyl-Carrier-Protein-Domäne) bezeichnet. Von dort können die Aminoacyl- oder Peptidylreste an das Nachbarmodul kondensiert werden. Diese Reaktion wird durch die Kondensationsdomäne (C-Domäne) katalysiert. Diese drei, die C-, die A- und die T-Domäne bilden üblicherweise die Grundeinheit der multimodularen NRPSs, wobei das erste Modul einer NRPS in der Regel keine C-Domäne enthält. Das letzte Modul einer NRPS enthält in der Regel eine Thioesterase- oder Terminationsdomäne (Te-Domäne) oder alternativ die Reduktasedomäne (R-Domäne), die für die Freisetzung des Syntheseproduktes verantwortlich ist. Dabei kann die Te-Domäne eine Übertragung auf ein Wassermolekül (Hydrolyse, führt zu linearen Produkten) oder auf eine funktionelle Gruppe des gerade hergestellten Peptides katalysieren (Amid- oder Esterknüpfung, führt zu zyklischen oder verzweigt zyklischen Produkten). Anstelle einer C-Domäne kann auch eine Cyclisierungsdomäne (Cy-Domäne) auftreten, die neben der Kondensation eine Cyclisierung des betreffenden Teils des Peptids bewirkt.

An Stellen, an denen eine modifizierte Aminosäure in das Peptid eingebaut wird, sind Modifikationsdomänen in das entsprechende Modul insertiert. Beispiele für Modifikationsdomänen sind die Epimerisierungsdomäne (E-Domäne), die N-Methylierungsdomäne (M-Domäne), die N-Formylierungsdomäne (F-Domäne) und die Oxidationsdomäne (Ox-Domäne).

Eine NRPS kann, muss aber nicht, auf mehr als eine enzymatische Untereinheit verteilt sein. In diesem Fall, der in Systemen bakteriellen Ursprungs die Regel ist, interagieren die entsprechenden Untereinheiten und übergeben die wachsende Peptidkette (z.B. besteht die gesamte Tyrocidin-NRPS aus drei Untereinheiten, den NRPS TycA, TycB und Tyc, die ein, drei bzw. sechs Module enthalten).

Es wurden bereits Versuche unternommen, durch den Austausch von Aminosäuren innerhalb von A-Domänen die Spezifität der Domäne zu beeinflussen (Deutsche Patentanmeldung Nr. 19909164.3-44). Auch der Austausch von Domänen zur Variation von bekannten Verbindungen wurde schon vorgeschlagen (EP-A-0 789 078).

Ein Problem bei solchen Austauschen war es immer, dass es keine klar definierten Grenzen oder Übergänge zwischen den einzelnen Domänen zu geben schien (Schneider et al. (1998), Mol. Gen. Genet. 257, S. 308-318). Zudem wurde zum Beispiel gefunden, dass C- und A-Domänen jeweils Selektivitäten aufweisen, die bei künstlicher Kombination in der Praxis zu Inkompatibilitäten und zum Stopp der gesamte Peptidsynthese führen können (Belshaw et al. (1999), Science 284, S.486-489).

Wir konnten nun zeigen, dass zwischen den einzelnen Domänen und/oder Modulen in einem Bereich von wenigen Aminosäuren Linker verbunden sind, die eine hohe Variabilität aufweisen. Die Linker tolerieren die durch gezielte Einfügung von Schnittstellen für Restriktionsendonukleasen auf DNA-Ebene bewirkten Veränderungen der Aminosäuresequenz, ohne die Funktion der Domänen oder Module zu beeinträchtigen. Die auf diese Weise erzeugten Peptidsynthetasen zeichnen sich gegenüber den auf herkömmliche Weise erzeugten NRPS durch eine hohe Aktivität aus bzw. sind überhaupt erst dazu in der Lage, die gewünschten Peptide zu synthetisieren.

Ebenso ist es mit dem beschriebenen Verfahren möglich, Module durch Fusion in die NRPS zu integrieren, die Actetat- bzw. Propionat-Einheiten in das Peptidrückgrat einbauen. Diese Einheiten leiten sich von Coenzym-A-Estern der Malonsäure oder einer α-substituierten, beispielsweise alkyl substituierten Malonsäure ab. Diese Module findet man in den Polyketidsynthasen (PKS), die analog NRPS modular aufgebaut sind und bei denen grundsätzlich das gleiche Syntheseprinzip der Thioesterbindung der Zwischenprodukte verfolgt wird (Staunton et al. (1997), Chem. Rev. 97, S.2611-2629). Hybride aus NRPS- und PKS-Modulen findet man auch in natürlichen Systemen, wie den Biosynthese-Enzymen für Yersiniabactin. Ein Modul ist dabei aus den Domänen Ketosynthase (KS) zur Bindungsknüpfung (entspricht der C-Domäne), Acyltransferase (AT) zur kovalenten Beladung des monomeren Bausteins als Thioester auf das Enzym (entspricht der A-Domäne) und eines Acyl-Carrier-Proteins (ACP) zur Bindung des Bausteins als Thioester an der Sulfhydrylgruppe des Kofaktors 4'-Phosphopantethein (entspricht der T-Domäne) aufgebaut. Zusätzliche reduktive Domänen entsprechen den Modifkationsdomänen bei NRPS, die schrittweise den nach der Kondensation entstehende β-Keto-Gruppe zur Hydroxylfunktion (Ketoreduktase, KR), α-β-ungesättigten Kette (Dehydratase, DH), oder zur vollständig gesättigten Kette (Enoylreduktase, ER) reduzieren können. Die Spezifität für Acetyl- bzw. Propionyl-Bausteine liegt bei der AT-Domäne, die entweder Malonyl-oder Methyl-Malonyl-CoA als Substrat erkennt.

Durch die Fusion von für singuläre Module bzw. Domänen kodierende Genfragmenten in den Bereichen, die für die Linker kodieren, z. B. durch die gezielte Einfügung von definierten Schnittstellen von Restriktionsendonukleasen in diese Linkerbereiche, wurde es überraschenderweise möglich, rekombinante NRPS zu synthetisieren, die ihrerseits Peptide vorherdefinierter Struktur synthetisieren können. Es wird hier also zum ersten Mal ein Verfahren beschrieben, durch das es möglich ist, mittels eines "modularen Molekülbaukastens" gezielt NRPS herzustellen, die zur Synthese von konstruierten Peptiden, vorzugsweise von solchen, von denen man eine vorteilhafte Wirkung kennt oder erwartet, herzustellen.

Konstruierte Peptide im Sinne der Erfindung, können sowohl bekannte Peptide und deren Derivate sein, als auch vorzugsweise Peptide, die z.B. durch Computer Aided Molecular Design oder ähnliche Verfahren entworfen wurden.

Bevorzugt ist auch eine Methode zur Biosynthese von Peptiden, bei der aufgrund der hier gemachten Ausführungen und der erfindungsgemäßen technischen Lehre, das rekombinante Gen bzw. die rekombinante Gene, die aus für Modulen kodierenden Genfragmenten zusammengesetzt werden und für entspechende erfindungsgemäße Peptidsynthetasen kodieren, nach für den Fachmann an sich bekannten Verfahren in einen Mikroorganismus, bevorzugterweise Bacillus subtilis, Escherichia coli, Saccharomyces cerevisiae oder Mikroorganismen der Gattung der Streptomyceten, integriert werden, z.B. als vektorkodierte Gene oder ins Chromosom integriert, um die gewünschte Peptidsynthetasen zu produzieren, die ihrerseits dann das gewünschte, maßgeschneiderte Peptid im verwendeten Mikroorganismus synthetisieren können.

Die Linkerbereiche zur Fusion befinden sich vorzugsweise an folgenden Postionen:
a) Der Linkerbereich zwischen einer T- und der folgenden Domäne, beispielsweise einer C-, E-, KS- oder Te-Domäne, hat eine Länge von 12 Aminosäuren und befindet sich zwischen den Aminosäuren 34 bis 45 (jeweils einschließlich) carboxyterminal des 4'-Phosphopantethein-bindenden Serins der Sequenz DxFFxxLGG(DH)S(IL) (Sequenzbezeichnung nach Marahiel et al. (1997), Chem. Rev. 97: S.2651-2673) der T-Domäne. Besonders bevorzugt zur Fusion ist der Bereich zwischen Aminosäure 38 und 39.
b) Der Linkerbereich zwischen einer A- und einer T-Domäne hat eine Länge von 9 Aminosäuren und befindet sich im Bereich Aminosäure 10-18 (jeweils einschließlich) carboxyterminal zu dem Lysin der Sequenz NGK(VL)DR (Sequenz A10 der A-Domäne (Sequenzbezeichnung nach Marahiel et al. (1997), Chem. Rev. 97: S.2651-2673)). Besonders bevorzugt zur Fusion ist der Bereich zwischen Aminosäure 16 und 17.
c) Der Linkerbereich zwischen einer C- und einer A-Domäne hat eine Länge von 23 Aminosäuren und befindet sich im Bereich Aminosäure 38-60 (jeweils einschließlich) aminoterminal zu dem Leucin der Sequenz L(TS)YxEL (Sequenz A1 der A-Domäne (Sequenzbezeichnung nach Marahiel et al. (1997), Chem. Rev. 97: S.2651-2673)). Besonders bevorzugt zur Fusion ist der Bereich zwischen Aminosäure 47 und 48.
d) Der Linkerbereich zwischen einer E- und einer C-Domäne hat eine Länge von 20 Aminosäuren und befindet sich im Bereich Aminosäure 9-28 (jeweils einschließlich) aminoterminal zu dem Serin der Sequenz SxAQxR(LM)(WY)xL (Sequenz C1 der C-Domäne (Sequenzbezeichnung nach Marahiel et al. (1997), Chem. Rev. 97: S.2651-2673)). Besonders bevorzugt zur Fusion ist der Bereich zwischen Aminosäure 20 und 21.

Die Erfindung betrifft neue NRPS gemäß der in Anspruch 1 gegebenen Definition. Ferner betrifft die Erfindung ein Verfahren zur Herstellung dieser NRPS umfassend die in Anspruch 18 definierten Maßnahmen.

Sollen Polyketid-synthasen (PKS) in die NRPS eingebaut werden so können weitere Linkerbereiche zur Fusion herangezogen werden. Diese Linkerbereiche befinden sich vorzugsweise an folgenden Positionen:
e) Der Linkerbereich zwischen einer T-Domäne und einer darauf folgenden KS-Domäne hat eine Länge von 12 Aminosäuren und befindet sich im Bereich zwischen den Aminosäuren 34 bis 45 (jeweils einschließlich) carboxyterminal des Serins der Sequenz DxFFxxLGG(DH)S(IL) der T-Domäne. Besonders bevorzugt zur Fusion ist der Bereich zwischen Aminosäure 38 und 39.
f) Der Linkerbereich zwischen einer ACP-Domäne und einer darauf folgenden Domäne hat eine Länge von 12 Aminosäuren und befindet sich im Bereich zwischen den Aminosäuren 34 bis 45 (jeweils einschließlich) carboxyterminal des 4'-Phosphopantethein-bindenden Serins der Sequenz (LI)GxDSL der ACP-Domäne. Besonders bevorzugt zur Fusion ist der Bereich zwischen Aminosäure 38 und 39.
g) Der Linkerbereich zwischen einer A- und einer ACP-Domäne kann generiert werden durch Fusion innerhalb des Bereiches der Aminosäuren 10-18 (jeweils einschließlich) carboxyterminal zu dem Lysin der Sequenz NGK(VL)DR der A-Domäne und innerhalb des Bereiches 46-77 Aminosäuren aminoterminal zu dem 4'-Phosphopantethein-bindenden Serin der Sequenz (LI)GxDSL der ACP-Domäne. Besonders bevorzugt zur Fusion ist der Bereich zwischen Aminosäure 16 und 17 carboxyterminal zu dem Lysin der Sequenz NGK(VL)DR der A-Domäne sowie der Bereich 46-77 Aminosäuren aminoterminal zu dem 4'-Phosphopantethein-bindenden Serin der Sequenz (LI)GxDSL der ACP-Domäne.

Vorzugsweise wird in dem DNA-Bereich, der für die betreffenden Linker kodiert, eine artifizielle Schnittstelle für eines der folgenden Restriktionsenzympaare eingebaut: Bam HI und Bgl II, sowie Xba I und Nhe I.

Der Vorteil der Verwendung dieser Schnittstellen ist, dass sie nach der Ligierung nicht mehr von den ursprünglich verwendeten Restriktionsenzymen erkannt werden. Die bevorzugten Enzympaare erzeugen kompatible Schnittstellen, die, wenn sie ineinander ligiert sind für beide Enzyme keine Restriktionssequenzen mehr zeigen.

Prinzipiell sind aufgrund der Variabilität dieses Bereiches jedoch keine Einschränkungen hinsichtlich der Verwendung anderer Restriktionsenzyme gegeben.

Bei dem Ausschneiden von Modulen, die Modifikationsdomänen enthalten, sollte die Lage dieser Domänen berücksichtigt werden. Liegt die Modifikationsdomäne zwischen einer A- und einer T-Domäne - wie das z.B. bei der Methylierungsdomäne der Fall ist - so ist keine Änderung gegenüber dem Ausschneiden eines einfachen CAT-Moduls notwendig:
C A M T |C A T (Schnittstelle |).

Liegt die Modifikationsdomäne hingegen hinter der T-Domäne eines Moduls, wie das z.B. bei der Epimerisierungsdomäne (E-Domäne), der Zyklisierungsdomäne (Z-Domäne) und der Reduktasedomäne (R-Domäne) der Fall ist, so sollte dies beim Fusionieren berücksichtigt werden, indem in diesem Fall, in dem Linkerbereich zwischen A- und T-Domäne fusioniert werden sollte.

Wir konnten zeigen, dass die T- und die folgende Domäne sich gegenseitig beeinflussen. Für die Praxis heißt das, dass es für eine optimale Synthese von Vorteil ist, wenn beim Fusionieren die auf die T_{xy}-Domäne folgende Domäne in ihrer Funktion der Domäne (xy) entspricht, die auch ursprünglich der T_{xy-}Domäne in ihrer natürlichen Struktur folgte. Folgte also auf eine T-Domäne eine C-Domäne, so handelt es sich um eine T_{c}-Domäne, die in Fusionsschritten wiederum mit einer C-Domäne fusioniert werden kann, z.B. C A T_{C} | C A T (Schnittstelle |).

Wird aber nach der T-Domäne eine Modifikationsdomäne eingebaut, z.B. eine E-Domäne, so sollte vorteilhafterweise die verwendete T-Domäne eine T_{E-}Domäne sein, das heißt eine T-Domäne, auf die in ihrer ursprünglichen Struktur auch eine E-Domäne folgte. Um dies sicherzustellen wird in einem solchen Fall vorteilhafterweise T- und E-Domäne zusammen ausgeschnitten: C A | T_{E} E C A T (Schnittstelle |).

In diesem Sinne sind dagegen T_{C}-, T_{Te}-, T_{KS}- und T_{z}-Domänen kompatibel und müssen in der Regel nicht gesondert berücksichtigt werden.

Wir konnten auch zeigen, dass das erste Modul einer mit einer C-Domäne beginnenden NRPS-Untereinheit natürlicherweise am Anfang eine variable Region von bis zu 40 Aminosäuren, üblicherweise 10 bis 15 Aminosäuren aufweist, diese Region ist wichtig für die Aktivität der Synthetase, insbesondere für die Reaktion mit der vorhergehenden NRPS-Untereinheit, von der die wachsende Peptidkette transferiert wird.

Überraschenderweise zeigte sich, dass auch einzelne Modifikationsdomänen in Konstrukten, die nur aus einem Modul bestehen, frei oder an eine Festphase gebunden, ihre Modifikationsfunktion erfüllen. Dadurch ist es möglich, Modifikationsgeneratoren zu bauen, die z.B. mittels einer Epimerisierungsdomäne L-Aminosäuren in D-Aminosäuren umwandeln. Die geeignete Gestaltung solcher Generatoren kennt der Fachmann an sich aus den bekannten Enzymreaktoren.

Die Modifikationsmodulkonstrukte bestehen im wesentlichen aus den für die umzusetzende Aminosäure affinen A- und T-Domänen, die mit der gewünschte Modifikationsdomäne und ggfls. einer Te-Domäne oder ggf. einer R-Domäne verbunden werden. Es können weitere Peptidsequenzen am Anfang oder am Ende angefügt werden, um die Wirksamkeit des Konstruktes, oder seine Handhabbarkeit, z.B. die Anbindung an eine feste Phase zu verbessern.

Es handelt sich bei den Modifikationsgeneratoren also um eine besondere Ausgestaltung der erfindungsgemäßen NRPS bzw. um die Anwendung des erfindungsgemäßen Verfahrens der Fusion einzelner Domänen auf ein einzelnes Modul.

Die Erfindung betrifft insbesondere ein Verfahren zur maßgeschneiderten Synthese artifizieller nicht-ribosomaler Peptidsynthetasen (NRPS), wobei a1) eine DNA-Sequenz ausgewählt wird, die für eine natürlich vorkommende Abfolge von Domänen oder Modulen kodiert, die eine vorbestimmte Abfolge von Aminosäuren eines vorbestimmten Peptids anlagern kann, a2) aus dieser DNA-Sequenz ein vorbestimmter Anteil mittels an sich bekannter Methoden entfernt wird, der für ein oder mehrere Domänen oder Module kodiert, die eine Aminosäure oder eine vorbestimmte Abfolge von Aminosäuren des Peptids anlagern kann, und a3) die verbliebenen DNA-Teilsequenzen zur gewünschten NRPS mittels an sich bekannter Methoden in den oben beschriebenen Linkerbereichen fusioniert werden.

Die Erfindung betrifft weiterhin insbesondere ein Verfahren zur maßgeschneiderten Synthese artifizieller nicht-ribosomaler Peptidsynthetasen (NRPS), wobei b1) eine DNA-Sequenz ausgewählt wird, die für eine natürlich vorkommende Abfolge von Domänen oder Modulen kodiert, die eine vorbestimmte Abfolge von Aminosäuren eines vorbestimmten Peptids anlagern kann, b2) aus dieser DNA-Sequenz ein vorbestimmter Anteil mittels an sich bekannter Methoden entfernt wird, der für ein oder mehrere Domänen oder Module kodiert, die eine Aminosäure oder eine vorbestimmte Abfolge von Aminosäuren des Peptids anlagern kann, und b3) die verbliebenen DNA-Teilsequenzen zusammen mit einer DNA-Sequenz, die für eine Domäne oder eine Abfolge von Domänen oder für ein Modul oder eine Abfolge von Modulen kodiert, die eine vorbestimmte Aminosäure oder eine vorbestimmte Abfolge von Aminosäuren eines Peptids anlagern kann, zur gewünschten NRPS mittels an sich bekannter Methoden in den oben beschriebenen Linkerbereichen fusioniert werden.

Eine Variante des erfindungsgemäßen Verfahrens besteht darin, dass für die Konstruktion rekombinanter NRPS zur Produktion eines definierten Peptides jeweils die DNA-Fragmente aus verscheidenen für NRPS kodierenden DNA-Abschnitten mittels PCR amplifiziert werden, die für die benötigten Domänen und/oder Module kodieren. In die zur PCR verwendeten Oligonukleotide können dabei Schnittstellen eingefügt werden, die eine leichte Verknüpfung der DNA-Fragmente durch Ligation erlauben. Die DNA-Fragmente werden dabei so ausgewählt, dass der Bereich ihrer Verknüpfungsstelle kodierend für die Linkerbereiche zwischen Domänen bzw. Modulen ist. Die Berücksichtigung dieser Linkerbereiche ist entscheidend für die Aktivität der so neu hergestellten NRPS. Die DNA-Fragmente können schrittweise in einen geeigneten Vektor eingebaut werden, z.B. pTZ18 oder pUC18 (Pharmacia, Freiburg, Best.Nr.: 27-4949-01), so dass schliesslich die für die Module bzw. Domänen kodierenden Fragmente in der gewünschten Reihenfolge miteinander verbunden im Vektor vorliegen.

So kann z.B. für die Ligation mehrerer Fragmente, die mit den PCR-Oligonukleotiden mit Schnittstellen für die Restriktionsendonukleasen Nhe I (5'-Ende) und Xba I (3'-Ende) versehen sind, folgendermassen verfahren werden.

Von dem Vektor pTZ18R ausgehend wird in einem Zyklus
1) mit Xba I hydrolytisch gespalten,
2) mit CIP Phosphatase dephosphoryliert und das gereinigte DNA-Fragment mit
3) dem jeweils nächsten PCR-Fragment, welches mit Xba I und Nhe I hydrolytisch gespalten worden war, mittels T4 DNA Ligase ligiert;
4) kompetente Escherichia coli-Zellen werden mit einem Aliquot des Ligationsansatzes transformiert;
5) von Kolonien, die nach Selektion auf Ampicillin erhaltenen wurden,
6) wird extrachromosomale DNA präpariert;
7) die gewünschte Plasmidkonstruktion, d.h. die Insertion des DNA-Fragmentes in das Plasmid in richtiger Orientierung, wird durch geeignete Restriktionsanalysen ermittelt.

Ein so erhaltenes Plasmid kann für die Insertion des nächsten DNA-Fragmentes verwendet werden.

Die so erhaltene rekombinante DNA wird dann nach dem Fachmann an sich bekannten Verfahren in einem geeigneten Organismus, vorzugsweise einem Mikroorganismus, bevorzugterweise in Escherichia coli, Bacillus subtilis, Saccharomyces cervisiae oder Mikroorganismen der Gattung Streptomyces ggfls. unter Verwendung anderer, geeigneter Vektoren exprimiert. Zur Modifikation der so produzierten rekombinanten NRPS mit dem Kofaktor 4'-Phosphopantethein kann dabei entweder im Mikroorganismus das Gen für eine 4'-Phosphopantethein-Transferase ko-exprimiert werden (Stachelhaus et al. (1998), J. Biol. Chem. 273: S.22773-22781), oder die rekombinanten NRPS können nach Isolierung in vitro mit einer 4'-Phosphopantethein-Transferase und Coenzym A modifiziert werden (Lambalot et al. (1996) Chem. & Biol. 3: S.923-926).

Die Isolierung der exprimierten Enzyme kann entweder nach dem Fachmann an sich ebenfalls bekannten Verfahren isoliert und in-vitro zur Synthese der gewünschten Peptide eingesetzt werden oder sie werden im Organismus belassen und die gewünschten Peptide werden in-vivo synthetisiert. Auf diese Weise können auch, insbesondere pharmakologisch wirksame Peptide in Eukaryontenzellen, insbesondere in Säugetierzellen und in Pflanzenzellen in-vivo synthetisiert werden.

Im wesentlichen kann also die Konstruktion auf der DNA-Ebene durchgeführt werden.

Die folgenden Beispiele dienen lediglich der Erläuterung der Erfindung und sollen deren Offenbarung in keiner Weise einschränken. Die Sequenzen der in diesen Beispielen verwendeten Oligonukleotiden sind im Anschluß an Beispiel 3 in Tabelle 2 aufgeführt.

### Beispiel 1: Herstellung künstlicher Peptidsynthetasen

Eine synthetische Peptidsynthetase wurde hergestellt, die nach dem erfindungsgemäßen Verfahren ein Peptidprodukt synthetisiert. Dazu wurde ein aus zwei Modulen bestehendes System zu einem aus drei Modulen bestehenden System verlängert. Die ersten beiden Module, TycA und ProCAT, entsprechen den ersten beiden Modulen der Tyrocidin-Peptidsynthetasen aus *Bacillus brevis* ATCC8185 (Mootz et al. (1997) J. Bacteriol. 179: S.6843-6850). TycA enthält eine A-Domäne mit Spezifität für Phenylalanin, eine T-Domäne und eine E-Domäne, die auf der T-Domäne gebundenes L-Phenylalanin in die D-Form umwandelt. ProCAT enthält eine C-Domäne, eine A-Domäne mit Spezifität für L-Prolin und eine T-Domäne. Unter Zugabe von ATP (10 mM), MgCl₂ (10 mM), L-Phenylalanin und L-Prolin (je 1 mM) in einem geeigneten Puffersystem (z. B. assay-Puffer: HEPES 50 mM, NaCl 100 mM, pH 8,0) produzierten TycA und ProCAT das Dipeptid D-Phe-Pro, welches als Thioester an ProCAT gebunden war. Eine sich anschließende, nicht enzymatisch katalysierte Reaktion führte zur Abspaltung des zyklischen D-Phe-Pro-Diketopiperazins. Durch Fusion auf genetischer Ebene wurden nun weitere Module XaaCAT nach dem erfindungsgemäßen Verfahren mit ProCAT verbunden, so dass aus 2 Modulen bestehende Enzyme der Art ProCAT-XaaCAT-Te ("-" entspricht der Fusionsstelle) entstanden, die die vorhergesagte Spezifität aufwiesen und zusammen mit TycA zur Synthese der vorhergesagten Tripeptide führten (Beispiele 1a und 1b).

### Beispiel 1a:

Das letzte Modul der Tyrocidin-Peptidsynthetase TycC, LeuCAT, besteht aus einer C-Domäne, einer A-Domäne mit Leucin-Spezifität und einer T-Domäne. Im direkten Anschluß folgt als letzte Komponente von TycC eine Te-Domäne. Das Genfragment für das Modul mitsamt der Te-Domäne, LeuCATTe, wurde nach dem erfindungsgemäßen Verfahren mit dem für ProCAT kodierenden Fragment fusioniert. Das so erhaltene Enzym ProCAT-LeuCATTe ("-" entspricht der Fusionsstelle) wies in der Aminoacyladenylatbildungsreaktion Spezifität für die Aminosäuren L-Prolin und L-Leucin auf Für die Aminoacyladenylatbildungsreaktion wurde die sogenannte ATP/PPi-Austauschreaktion durchgeführt. Dazu wurden in Eppendorf-Reaktionsgefäßen jeweils die zu testenden Aminosäuren und ATP vorgelegt und auf 37°C präinkubiert. Dazu wurde ein ebenfalls auf 37°C präinkubiertes Gemisch aus Enzym, nicht-radioaktiv markiertem und radioaktiv markiertem PPᵢ und MgCl₂ (in assay Puffer) pipettiert. Das Reaktionsgemisch wurde für 15 min bei 37°C inkubiert, anschließend auf Eis transferiert und mit 0,5 mL eiskalter Terminationslösung versetzt. Der Ansatz wurde vortexiert und 1 min auf Eis inkubiert. Anschließend wurde die Aktivkohle durch Zentrifugation (1 min; 13,000 Upm) pelletiert, zweimal durch Resuspension in 0,8 mL H₂O und erneuter Zentrifugation gewaschen, in 0,5 mL H₂O resuspendiert, in 20 mL Szintillationsmeßfläschchen überführt und mit 4 mL Szintillationsflüssigkeit Rotiszint Eco Plus versetzt. So konnte die Zählrate der Probe in einem Szintillationszähler gemessen werden.

| | | |
|---|---|---|
| **ATP-PP**_{**i**}**-Austauschreaktion** | Aminosäure 10 mM | 10 µL |
| | ATP 100 mM | 5 µL |
| | Enzym | 50 pmol |
| | MgCl₂ 1M | 1 µL |
| | Natriumpyrophosphat 50 mM | 0,2 µL |
| | [³²P]-Pyrophosphat | 0,15 µCi |
| | assay Puffer pH 8,0 add. | 100 µL |
| Terminationslösung | Natriumpyrophosphat | 100 mM |
| | Perchlorsäure | 560 mM |
| | Aktivkohle (Norit A) | 1,2 % (w/v) |

Wenn ProCAT-LeuCATTe mit TycA inkubiert wurde, konnte die Produktion des Tripeptides D-Phe-Pro-Leu nachgewiesen werden. Dazu wurden in einem Eppendorf-Reaktiongefäß in einem Gesamtvolumen von 100 µL jeweils 50 pmol TycA und ProCAT-LeuCATTe in assay-Puffer mit 10 mM MgCl₂, 5 mM ATP, 1 mM L-Phe, 1 mM L-Pro und 1 mM L-Leu für 2 Stunden bei 37°C inkubiert. Nach Stoppen der enzymatischen Reaktion durch Zugabe von 50 µL n-Butanol/Chloroform (4:1) wurde der Ansatz unter vermindertem Druck bis zur Trockne eingeengt. Das trockne Pellet wurde mit 10 % HPLC-Puffer B (0,04% HCOOH in Methanol) in HPLC-Puffer A (0,05 % (v/v) HCOOH in H₂O) versetzt, gelöst und der klare Überstand nach Zentrifugation abgenommen. Dieser Überstand wurde nun zur Produkttrennung und -identifizierung mit einer HPLC/MS-Anlage (feste Phase 250/3-Nucleosil-C18 reversed phase-Säule von Macherey&Nagel, flüssige Phase Gradient HPLC -Puffer A/B: 0 min, 10 % HPLC-Puffer B; 1 min, 30 % HPLC-Puffer B; 20 min, 100 % HPLC-Puffer B; 30 min 100 % HPLC-Puffer B; 35 min, 10 % HPLC-Puffer B; 50 min, 10 % HPLC-Puffer B; Flußrate 0,3 mL/min) verwendet. Das Produkt D-Phe-Pro-Leu konnte durch sein Laufverhalten, welches identisch mit einem chemisch synthetisierten Standard von D-Phe-Pro-Leu war, und seine Masse ([M+H]⁺-Peak bei 376 Da) nachgewiesen werden. Die Geschwindigkeit der Bildung von D-Phe-Pro-Leu wurde mit 2,1 min⁻¹ bestimmt. (s. unten für die Konstruktion der Expressionsplasmide und Isolierung der rekombinanten NRPSs)

### Beispiel 1b:

Das fünfte und vorletzte Modul der Tyrocidin-Peptidsynthetase TycC, OrnCAT, besteht aus einer C-Domäne, einer A-Domäne mit Ornithin-Spezifität und einer T-Domäne. Das Genfragment für dieses Modul wurde nach dem erfindungsgemäßen Verfahren mit dem für ProCAT kodierenden Fragment fusioniert. Das so erhaltene Enzym ProCAT-OrnCAT ("-" entspricht der Fusionsstelle) wies Spezifität für die Aminosäuren L-Prolin und L-Ornithin auf, und produzierte bei Inkubation mit TycA (wie oben, zusätzliche Zugabe von 1 mM L-Ornithin) das Tripeptid D-Phe-Pro-Orn. Dieses wurde jedoch nicht enzymkatalysiert abgespalten.

In einem weiteren Schritt wurde das Genfragment, welches für die Te-Domäne von TycC kodiert, nach dem erfindungsgemäßen Verfahren fusioniert, so daß das Enzym ProCAT-OrnCAT-Te ("-" entspricht der Fusionsstelle) erhalten wurde. Dieses Enzym wurde mit TycA inkubiert (wie oben, zusätzliche Zugabe von 1 mM L-Ornithin) und produzierte das Tripeptid D-Phe-Pro-Orn, welches auch vom Enzym abgespalten wurde. Dazu wurden in einem Eppendorf-Reaktiongefäß in einem Gesamtvolumen von 100 µL jeweils 50 pmol TycA und ProCAT-OrnCAT-Te in assay-Puffer mit 10 mM MgCl₂, 5 mM ATP, 1 mM L-Phe, 1 mM L-Pro und 1 mM L-Orn für 2 Stunden bei 37°C inkubiert. Stoppen der Reaktion, Aufarbeitung und HPLC/MS-Analyse erfolgte wie in Beispiel 1a beschrieben. Das Produkt D-Phe-Pro-Orn wurde mit seiner Masse ([M+H]⁺⁻Peak bei 377 Da) nachgewiesen. Die Geschwindigkeit der Bildung von D-Phe-Pro-Orn wurde mit 0,15 min⁻¹ bestimmt. (s. unten für die Konstruktion der Expressionsplasmide und Isolierung der rekombinanten NRPSs)

### Konstruktion der Expressionsplasmide und Isolierung der rekombinanten NRPS für Beispiele 1a und 1b:

Alle Genfragmente wurden mit Vent-Polymerase von New England Biolabs (Best.-Nr.: 254S, Schwalbach/Taunus, Deutschland, gemäß den Herstellerangaben eingesetzt) aus chromosomaler DNA von *Bacillus brevis* ATCC8185 amplifiziert. Für das für TycA kodierende Genfragment wurden die Oligonukleotide Seq ID-NO:1 und Seq ID-NO:2 verwendet, für das für ProCAT kodierende Genfragment die Oligonukleotide Seq ID-NO:3 und Seq ID-NO:4 , für das für LeuCATTe kodierende Genfragment die Oligonukleotide Seq ID-NO:5 und Seq ID-NO:6, für das für OrnCAT kodierende Genfragment die Oligonukleotide Seq ID-NO:7 und Seq ID-NO:8, für das die Te-Domäne kodierende Genfragment die Oligonukleotide Seq ID-NO:9 und Seq ID-NO:6. Die PCR-Amplifikate wurden mit dem "QIAquick-spin PCR purification"-System (Qiagen; Hilden, Deutschland, Katalognr.: 28104) gereinigt und ihre Enden mit den Restriktionsendonukleasen *Nco* I und *Bam* HI (TycA, ProCAT), *Bam* HI (OrnCAT, LeuCATTe) sowie *Bam* HI und *Bgl* II (Te) hydrolytisch gespalten. Die jeweiligen Erkennungssequenzen für die Restriktionsendonukleasen waren in den verwendeten Oligonukleotiden enthalten (alle Restriktionsendonukleasen von Amersham/Buchler; Braunschweig, Deutschland; Nco I: Best.-Nr. E1160Z; Bam HI: Best.-Nr. E1010Y und Bgl II: Best.-Nr.1021Y). Die für TycA und ProCAT kodierenden Genfragmente wurden in den mit Nco I und Bam HI hydrolytisch gespaltenen Vektor pQE60 (Qiagen; Hilden, Deutschland, Best.-Nr.: 33603) mit T4 DNA Ligase (Amersham/Buchler, Braunschweig, Deutschland, E70005Y) ligiert. Nach Transformation von E. coli XL1Blue mit dem Ligationsansatz und Selektion auf Festmedium LB mit Ampicillin (100 µg/mL) als Selektionsantibiotikum und anschließender Inkubation über Nacht bei 37°C wurden Ampicillin-resistente Transformanten erhalten. Aus diesen Transformanten wurde jeweils Plasmid-DNA isoliert, welche anschließend durch Restriktionsanalyse (Verwendung der Enzyme Nco I, Bam HI, Hind III und Ava I) auf die Identität der gewünschten Konstruktion geprüft wurde. So konnten die Plasmide pTycA und pProCAT erhalten werden. Das Plasmid pProCAT wurde abermals mit Bam HI hydrolytisch gespalten, mit CIP-Phosphatase (New England Biolabs, Schwalbach/Taunus, Deutschland, Best.-Nr.: 290S) desphosphoryliert, mit dem "QIAquick-spin PCR purification"-System gereinigt und als Vektor zur Klonierung der hydrolytisch gespaltenen PCR-Fragmente für LeuCATTe und OrnCAT (s.o.) (Ligation mit T4 DNA Ligase) eingesetzt. Nach Transformation von E. coli XL1 Blue mit dem Ligationsansatz und Selektion auf LB mit Amipicillin (100 µg/mL) wurden Ampicillin resistente E. coli Kolonien erhalten. Diese wurden zur Plasmid-DNA Isolierung eingesetzt. Nach Restriktionsanalyse dieser Plasmide (Verwendung der Enzyme Nco I, Bam HI, Hind III und Ava I) wurden die Plasmide pProCAT-LeuCATTe und pProCAT-OrnCAT erhalten. Die richtige Orientierung der für LeuCATTe und OrnCAT kodierenden Fragmente wurde durch Restriktions-analyse mit der Restriktionsendonuklease Hind III verifiziert (Amersham / Buchler; Braunschweig, Deutschland, Best.-Nr. E1060Z). Das Plasmid pProCAT-OrnCAT wurde mit Bgl II abermals hydrolytisch gespalten, mit CIP-Phosphatase dephosphoryliert, mit dem "QIAquick-spin PCR purification"-System gereinigt und als Vektor zur Ligation mit dem hydrolytisch gespaltenen PCR-Fragment für die Te-Domäne (s.o.) eingesetzt (Ligation mit T4-DNA Polymerase). Nach Transformation von E.coli XL1Blue mit dem Ligationsansatz, Selektion von Ampicillin-resistenten Transformanten auf festem LB-Medium mit Ampicillin (100 µg/mL), Präparation von Plasmid-DNA aus diesen Stämmen und Analyse dieser Plasmide mittels Restriktionsenzymen (Verwendung der Enzyme Nco I, Bam HI, Hind III und Ava I) wurde das Plasmid pProCAT-OrnCAT-Te erhalten. Die richtige Orientierung des für die Te-Domäne kodierenden Fragmentes wurde durch Restriktionsanalyse mit der Restriktionsendonuklease Hind III verifiziert.

Die erhaltenen Plasmide wurden anschließend jeweils zur Transformation des *E. coli* Expressionsstammes *E. coli* BL21/pREP4-gsp (Stachelhaus et al. (1998), J. Biol. Chem. 273: S.22773-22781) eingesetzt. Dieser Stamm ermöglichte die Koexpression mit dem Gen gsp, das für die 4'-Phosphopantethein-Transferase Gsp kodiert, welche die posttranslationale Modifikation von Peptidsynthetasen mit dem Kofaktor 4'-Phoshopantethein katalysiert. Der Vektor pREP4-gsp verleiht Resistenz gegen Kanamycin, so dass nun Transformanten mit Kanamycin (25 µg/mL) und Ampicillin (100 µg/mL) selektiert wurden. Die so erhaltenen Stämme BL21/pREP4-gsp/pTycA, BL21/pREP4-gsp/pProCAT, BL21/pREP4-gsp/pProCAT-LeuCATTe, BL21/pREP4-gsp/pProCAT-OrnCAT und BL21/pREP4-gsp/pProCAT-OrnCAT-Te wurden wie beschrieben (Stachelhaus et al. (1998), J. Biol. Chem. 273: S.22773-22781) in Flüssigmedium zur Proteinproduktion eingesetzt und wie beschrieben mittels Affinitätschromatographie isoliert (Stachelhaus et al. (1998), J. Biol. Chem. 273: S.22773-22781).

Eine genauer Beschreibung dieser Vorgehensweise findet sich auch in Beispiel 1c).

### Beispiel 1c: A-T-Fusionen als Mittel zur Darstellung von Hybridpeptidsynthetasen

Dieses Beispiel beschreibt die Verwendung der Fusionsstelle zwischen A- und T-Domänen als Mittel zur Modulfusion und zur Herstellung von maßgeschneiderten Hybridpeptidsynthetasen, die die geplanten neuen Peptidprodukte synthetisieren.

### Klonierung der Gene für die Hybridpeptidsynthetasen

Ein 1613 bp DNA-Fragment wurde mit PCR und den DNA-Oligonukleotiden Seq ID-NO:35 und Seq ID-NO:36 aus chromosomaler DNA von *Bacillus licheniformis* ATCC 10716 amplifiziert. Das Fragment wurde mit dem "QIA quick spin purification kit" gereinigt und mit Hilfe der Restriktionsendonukleasen Nco I und Pst I hydrolytisch gespalten (37°C, 16 h).

Ein 4686 bp DNA-Fragment wurde per PCR mit den Oligonukleotiden Seq ID-NO:37 und Seq ID-NO:38 aus dem Plasmid pTycA (siehe Beispiel 1a) amplifiziert. Das amplifizierte Fragmentes enthält DNA-Sequenz des pQE-Vektors sowie Teile des tycA-Gens aus *Bacillus brevis* ATCC 8185. Das Fragment wurde mit dem "QIA quick spin purification kit" gereinigt und mit den Restriktionsendonukleasen *Nco* I und *Pst* I hydrolytisch gespalten (37°C, 16 h) und nachfolgend für eine Stunde bei 37°C mit Alkalischer Phosphatase inkubiert. Templat-DNA wurde anschließend durch Inkubation mit der Restriktionsendonuklease *Dpn* I hydrolytisch gespalten (37°C, 30 min).

Die beiden beschriebenen Fragmente wurden nachfolgend mit T4-DNA-Ligase ligiert (16°C, 16 h). E. coli XL1 Blue wurde mit einem Zehntel des Ligationsansatzes (10 µL) transformiert. Transformanten wurden auf 2xYT Agar-Platten (Ampicillin 100 µg/mL) selektiert. Von 48 Transformanten, die Ampicillin-Resistenz aufwiesen, wurden Plasmidpräparationen durchgeführt. 5 Transformanten enthielten Plasmide mit einer Größe von ca. 6.5 kbp. Die korrekte Insertion des 1605 bp-DNA-Fragmentes wurde durch hydrolytische Spaltung der Plasmid-DNA mit Restriktionsendonukleasen sowie durch terminale Sequenzierung bestätigt. Das erhaltene Plasmid wurde als p[A_{Ile}]_{*bac*A1}-[TE]_{*tyc*A} bezeichnet.

p[A_{Ile]*bac*A1}-[TE]_{*tyc*A} wurde durch hydrolytische Spaltung mit den Restriktionendonukleasen *Pst* 1 und *Bam* Hl hydrolytisch gespalten und durch Agarose-Gelelektrophorese von dem ebenfalls entstandenen 1704 bp DNA-Fragment abgetrennt. Das 5030 bp-DNA-Fragment wurde isoliert, mittels "QIA quick spin purification kit" gereinigt und anschließend mit Alkalischer Phosphatase modifiziert (37°C, 1 h). Ein 4131 bp DNA-Fragment wurde per PCR mit den Oligonukleotiden Seq ID-NO-39 und Seq ID-NO:40 aus chromosomaler DNA von *Bacillus brevis* ATCC 8185 amplifiziert. Das Fragment wurde mit den Restriktionsendonukleasen *Pst* I und *Bam* Hl hydrolytisch gespalten (37°C, 16 h). Beide DNA-Fragmente wurden T4-Ligase ligiert (16°C, 16 h). E. coli XL1Blue wurde mit einem zehntel des Ligationsansatzes (10 µL) transformiert. Transformanten wurden auf 2xYT Agar-Platten (Ampicillin 100 µg/mL) selektiert. Von 24 Transformanten, die Ampicillin-Resistenz aufwiesen, wurden Plasmidpräparationen durchgeführt. 3 Transformanten enthielten Plasmide einer Größe von ca. 9 kbp. Die korrekte Insertion des 4125 bp-DNA-Fragmentes wurde durch hydrolytische Spaltung der Plasmid-DNA mit Restriktionsendonukleasen sowie durch terminale Sequenzierung mit pQE-Standard-Sequenzierung-Oligonukleotiden bestätigt.

Das erhaltene Plasmid wurde als p[A_{Ile}]_{*bac*A1}-[TCA_{Leu}TTe]_{*tyc*C5-6} bezeichnet.

p[A_{Ile}]_{*bac*A1}-[TCA_{Leu}TTe]_{*tyc*C5-6} wurde zur Amplifikation eines 6035 bp DNA-Fragmentes per PCR mit den Oligonukleotide Seq ID-NO:41 und Seq ID-NO:42 benutzt. Das erhaltene DNA-Fragment wurde mit dem "QIA quick spin purification kit" gereinigt und mit Hilfe der Restriktionsendonuklease *Pst* I hydrolytisch gespalten (37°C, 16h). Zur hydrolytische Spaltung der Templat-Plasmid-DNA wurde anschließend eine Stunde bei 37°C mit *Dpn* I inkubiert. Mit Hilfe der T4-Ligase wurde das DNA-Fragment intramolekular religiert. E.coli XL1 Blue wurden mit einem zehntel des Ligationsansatzes (10 µL) transformiert. Transformanten wurden auf 2xYT Agar-Platten (Ampicillin 100 µg/mL) selektiert. Von 24 Transformanten, die Ampicillin-Resistenz aufwiesen, wurden Plasmidpräparationen durchgeführt. 12 enthielten, wie durch hydrolytische Spaltung mit Restriktionsendonukleasen gezeigt werden konnte, das gewünschte Plasmid P[AIₗₑ]_{*bac*A1}-[TTe]_{*tyc*C5-6} (6029 bp).

p[AIₗₑ]_{bacA1}-[TTe]_{*tyc*C5-6} wurde durch hydrolytische Spaltung mit den Restriktionendonukleasen *Pst* I und *Hpa* I hydrolytisch gespalten. Das 6026 bp-DNA-Fragment wurde mit dem "QIA quick spin purification kit" gereinigt und anschließend mit Alkalischer Phosphatase behandelt (37°C, 1 h). Mit Hilfe der Oligonukleotide Seq ID-NO:43 und Seq ID-NO:44 wurde per PCR ein 3117 bp DNA-Fragment aus chromosomaler DNA von *Bacillus brevis* ATCC 8185 amplifiziert. Das DNA-Fragment wurde mit dem "Qia quick spin purification kit" gereinigt und anschließend mit den Restriktionsendonukleasen *Pst* I und *Hpa* I hydrolytisch gespalten (37°C, 16 h). Beide DNA-Fragmente wurden dann mit T4 -Ligase ligiert. E.coli XL1 Blue wurde mit einem zehntel des Ligationsansatzes (10 µL) transformiert. Transformanten wurden auf 2xYT Agar-Platten (Ampicillin 100 µg/mL) selektiert. Von 24 Transformanten, die Ampicillin-Resistenz aufwiesen, wurden Plasmidpräparationen durchgeführt. 2 enthielten, wie durch hydrolytische Spaltung mit Restriktionsendonukleasen *Hpa* I und *Pst* I gezeigt werden konnte, ein Insert von 3117 bp. Zur Verifizierung der korrekten Insertion wurden die Fusionsstellen im Bereich der *Hpa* I und *Pst* I-Schnittstelle sequenziert. Das enstandene Plasmid wurde als P[A_{Ile}]_{bacA1}-[TCA_{Phe}]_{*tyc*B2}-[TTe]_{*tyc*C6} bezeichnet.

### Expression der rekombinanten Hybrid-Gene von p[A_{Ile}]_{bacA1}- [TCA_{Leu}TTe]_{tycC5-6} und p[A_{Ile}]_{bacA1}-[TCA_{Phe}]_{tycB2}-[TTe]_{tycC6}

Jeweils 1µL der konstruierten Plasmide p[A_{Ile}]_{*bac*A1}-[TCA_{Leu}TTe]_{*tyc*C5-6} und p[A_{Ile}]_{bacA1}-[TCA_{Phe}]_{tycB2}-[TTe]_{*tycC*6} wurden zur Transformation von kompetentem *E. coli* BL21/pREP4-gsp eingesetzt. Transformanten wurden jeweils auf 2xYT-Agarplatten (Ampicillin 100 µL/mL und Kanamycin 25 µL/mL) selektiert. Von einer Kolonie wurde jeweils eine 5 mL Kultur 2xYT Flüssigmedium (Ampicillin 100 µL/mL und Kanamycin 25 (µL/mL) inokuliert. Diese Kulturen wurden jeweils für 16 h bei 37°C unter Schütteln inkubiert. Jeweils 1 mL der Kulturen wurde verwendet um einen Glycerol-Stocks des rekombinanten Stammes anzufertigen, welcher bei -80°C zur Lagerung tiefgefroren wurde. 4 mL der Kulturen wurden benutzt, um jeweils 400 mL des gleichen Mediums anzuimpfen. Die Zellen wurden bei 30°C unter 250 Upm für 3-4 Stunden inkubiert. Nachdem sie jeweils eine optische Dichte von 0.7 (OD₆₀₀) erreicht hatten, wurde die Transkription der rekombinanten Gene durch die Zugabe von IPTG (Endkonzentration 200 µM) induziert. Die Zellen wurden jeweils für weitere 1,5 h angezogen, bevor sie geemtet wurden.

Die Überproduktion der rekombinanten Proteine [A_{Ile}]_{BacA1}-[TCA_{Leu}TTe]_{TycC5-6} (Protein kodiert auf Plasmid p[A_{Ile]*bac*A1}-[TCA_{Leu}TTe]_{*tyc*C5-6}) und [A_{Ile}]_{BacA1-}[TCA_{Phe}]_{TycB2}-[TTe]_{TycC6} ("-" entspricht der Fusionsstelle) (Protein kodiert auf Plasmid p[A_{Ile}]_{*bac*A1}-[TCAₚₕₑ]_{*tyc*B2}-[TTe]_{*tyc*C6)} wurde überprüft durch SDS-PAGE, indem Protein-Proben, die vor und nach IPTG-Induktion genommen wurden, verglichen wurden. Im Rohextrakt von BL21/pREP4-gsp/p[A_{Ile}]_{*bac*A1-}[TCA_{Leu}TTe]_{*t*y*c*C5-6} und BL21/pREP4-gsp/p[AIₗₑ]_{*bac*A1}-[TCA_{Phe}]_{*tyc*B2}-[TTe]_{*tyc*C6} konnte jeweils die Überproduktion eines Proteins von ca. 215 kDa beobachtet werden.

### Reinigung der rekombinanten Proteine [A_{Ile}]_{BacA1}-[TCA_{Leu}TTe]_{TycC5-6} und [A_{Ile}]_{BacA1}-[TCA_{Phe}]_{TycB2}-[TTe]_{TycC6}

800 mL Kulturen von BL21/pREP4-gsp/p[A_{Ile}]_{*bac*A1}-[TCA_{Leu}TTe]_{*tyc*C5-6} und BL21/pREP4-gsp/p[A_{Ile}]_{*bac*A1}-[TCA_{Phe}]_{*tyc*B2}-[TTe]_{*tyc*C6} wurden zentrifugiert (5000 Upm, 5 Minuten) und das erhaltene Zellpellet anschließend resuspendiert in 30 mUL Kultur Puffer A (50 mM HEPES, 300 mM NaCL, pH 8,0). Die Zellen wurden direkt weiterverwendet oder bis zur Weiterverwendung bei ―20 °C tiefgefroren. Die Zellen wurden durch zwei Passagen durch eine *French-Press* (Arbeitsdruck 12000 PSI) aufgebrochen. Anschließend wurden unlösliche Bestandteile durch Zentrifugation bei 15000 Upm für 15 Minuten abgetrennt. Der klare Überstand wurde mit 1% (v/v) Puffer B versetzt (50 mM HEPES, 300 mM NaCl, 250 mM Imidazol, pH 8,0). Die Proteinlösung wurde an einer FPLC auf eine vorher mit 1% Puffer B äquilibrierte Ni²⁺-NTA-Agarose-Säule (Qiagen) aufgetragen. Die Flußrate betrug 0,75 mL/min. Nachdem die Absorption (280 nm) nach Auftrag der Proteinlösung wieder auf Anfangsniveau gesunken war wurde ein linear ansteigender Gradient des Puffers B angewandt (30 Minuten auf 30 % Puffer B, 40 Minuten auf 100 % Puffer B). Eluate wurden in 2 mL-Fraktionen gesammelt. Die rekombinanten Proteine [A_{Ile}]_{BacA1}-[TCA_{Leu}TTe]_{TycC5-6} und [A_{Ile}]_{BacA1}-[TCA_{Phe}]_{TycB2}-[TTe]_{TycC6} eluierten bei Puffer B-Konzentrationen von ca. 5-10 %.

Fraktionen, die Protein [A_{Ile}]_{BacA1}-[TCA_{Leu}TTe]_{TycC5-6}, beziehungsweise [A_{Ile}]_{BacA1}-[TCA_{Phe]TycB2}-[TTe]_{TycC6} enthielten, wurden mittels Bradford-Test bei 595 nM detektiert, vereint und für 16 h gegen Assay-Puffer dialysiert (50 mM HEPES, 100 mM NaCl, 10 mM MgCl₂, 5 mM DTT). Anschließend wurde die Konzentration der dialysierten Proteinlösung nochmals bestimmt.

Aus 1 L Zellkultur konnten ca. 5 mg rekombinante Proteine [A_{Ile}]_{BacA1-}[TCA_{Leu}TTe]_{TycC5-6,} beziehungsweise [A_{Ile}]_{BacA1}-[TCA_{Phe}]_{TycB2}-[TTe]_{TycC6} erhalten werden, die, wie über SDS-PAGE verifiziert werden konnte, eine Reinheit von ca. 95 % aufwiesen.

### Enzymatische Aktivität der rekombinanten Proteine [A_{Ile}]_{BacA1}-[TCA_{Leu}TTe]_{TyC5-6} und [A_{Ile}]_{BacA1}-[TCA_{Phe}]_{TycB2}-[TTe]_{TycC6}

### ATP-PPᵢ-Austauschreaktion:

Die Spezifität der rekombinanten Proteine [A_{Ile}]_{BaCA1}-[TCA_{Leu}TTe]_{TycC5-6} und [A_{Ile]BacA1}-[TCA_{Phe}]_{TycB2}-[TTe]_{TycC6} bezüglich der Adenylierung von Aminosäuren wurde indirekt bestimmt durch die Inkorporation ³²P-markierten PPᵢ in ATP während der enzymatisch katalysierten Rückreaktion. Das Protokoll ist in Beispiel 1 a beschrieben.

Das Protein [A_{Ile}]_{BacA1}-[TCA_{Leu}TTe]_{TycC5-6} zeigte eine Spezifität für Isoleucin und Leucin. Auch Valin wurde zu einer Rate von 30 % bezogen auf die Leucin-Aktivierung aktiviert. Das Protein [A_{Ile}]_{BacA1}-[TCA_{Phe}]_{TycB2}-[TTe]_{TycC6} zeigte eine Tryptophan-, Phenylalanin- und Leucin-Spezifität. Tryptophan wurde dabei ebenfalls von der Phenylalanin A-Domäne aktiviert.

### Thioesterbindung:

Die Fähigkeit, die Substrataminosäure kovalent als Thioester zu binden, wurde über die Beladungsreaktion bestimmt. Dabei kann, wenn die Substrataminosäure ¹⁴C-markiert verwendet wird, die Inkorporation von Radioaktivität in präzipitiertes Protein als Maß für diese Beladung gelten.

50 pmol Protein [A_{Ile}]BacA1-[TCA_{Leu}TT]_{TycC5-6,} beziehungsweise [A_{Ile}]_{BacA1-}[TCA_{Phe}]_{TycB2}-[TTe]_{TycC6} wurden inkubiert mit 2 mM ATP, Assay-Puffer und 150 pmol der jeweiligen ¹⁴C-markierten Substrataminosäure. Nachdem 10 Minuten bei 37°C inkubiert wurde, wurden Proteine durch die Zugabe von 10% TCA gefällt, durch Zentriguation (30 min, 13000 Upm) pelletiert und das Pellet gewaschen (1x 0,8 mL 10% TCA). Das in 100% Ameisensäure gelöste Proteinpellet wurde im Szintillationszähler auf die Inkorporation von Radioaktivität getestet.

Die Proteine [A_{Ile}]_{BacA1}-[TCA_{Leu}TTe]_{TycC5-6} und [A_{Ile]BacA1}-[TCA_{Phe}]_{TycB2}-TTe]_{TycC6} zeigten jeweils eine signifikante Aktivität in der Thioesterbindung mit den im ATP-PPᵢ-Austausch aktivierten Substrataminosäuren. Protein [A_{Ile}]_{BacA1-}[TCA_{Leu}TTe]_{TycC5-6} konnte Isoleucin und Leucin binden. Protein [A_{Ile}]_{BacA1-}[TCA_{Phe}]_{TycB2}-[TTe]_{TycC6} konnte Isoleucin sowie Tryptophan und Phenylalanin binden.

### Produktbildung:

Produktbildung am Enzymtemplat wurde durchgeführt durch Inkubation der Proteine [A_{Ile}]BacA1-[TCA_{Leu}TTe]_{TycC5-6}. beziehungsweise [A_{Ile}]_{BacA1-}[TCA_{Phe}]_{TycB2}-[TTe]_{TycC6} mit den jeweiligen Substrataminosäuren (1 mM; siehe ATP-PPᵢ-Austauschreaktion) und ATP in Assay-Puffer. Die Identifikation von Reaktionsprodukten erfolgte über HPLC und HPLC-MS. Retentionszeiten von identifizierten Produkten wurden mit chemischen Standardprodukten (bezogen von Bachem, Heidelberg) verglichen.

50 pmol Protein [A_{Ile]BacA1}-[TCA_{Leu}TTe]_{TycC5-6}, beziehungsweise [A_{Ile]BacA1-}[TCA_{Phe]TycB2}-[TTe]_{TycC6} wurden jeweils mit 1 mM Substrataminosäure, 1 mM ATP in einem Gesamtvolumen Assay-Puffer von 100 µL für 2 h bei 37°C inkubiert. Die Reaktionen wurden jeweils durch Zugabe von 100 µL Butanol abgestoppt. Ausgefallene Proteine wurden mit einer Pipettenspitze abgetrennt bevor jeweils die gesamten Lösungen im Rotationsverdampfer unter verminderten Druck bis zur Trockne eingeengt wurde. Es wurde in jeweils 100 µL 10 % Methanol aufgenommen und ein Zehntel dieses Volumens für die HPLC, beziehungsweise HPLC-MS-Analytik eingesetzt. Die HPLC-Analytik erfolgte mittels eines Hewlett Packard 1100 HPLC-Systems und einer Säule Nucleosil C18 3/120 3250 (Macerey&Nagel, 3x250 mm, Porengröße 120 Angström, Partikelgröße 3 mm). Die Detektion erfolgte mit einem UV-Detektor bei 210 nm. Folgender Gradient wurde verwendet: 0 min 10% HPLC-Puffer B; 1 min 30 % HPLC-Puffer B; 20 min 100 % HPLC-Puffer B; 30 min 100 % HPLC-Puffer B; 35 min 10 % HPLC-Puffer B; 50 min 10 % HPLC-Puffer B; wobei die Flußrate 0,35 mL/min betrug (HPLC-Puffer A: H₂O mit 0,05 Volumenprozent Ameisensäure, HPLC-Puffer B: Methanol mit 0,45 Volumenprozenz Ameisensäure).

Protein [A_{Ile}]_{BacA1}-[TCA_{Leu}TTe]_{TycC5-6} bildete in Abhängigkeit der Substrataminosäuren Isoleucin und Leucin das Dipeptid lsoleucinyl-Leucin mit einer Geschwindigkeit von 0.3 Moleküle pro Minute.

Das Protein [A_{Ile}]_{BacA1}-[TCA_{Phe}]_{TycB2}-[TTe]_{TycC6} stellte unter Verwendung der Substrataminosäuren Isoleucin und Phenylalanin das Dipeptid Isoleucinyl-Phenylalanin mit einer Geschwindigkeit von 0.5 pro Minute dar. Ein Austausch der Substrataminosäuren von Phenylalanin nach Tryptophan senkte die entsprechende Dipeptidbildung von Isoleucinyl-Tryptophan auf eine Geschwindigkeit von unter 0.02 pro Minute ab.

### Beispiel 1d: Rolle der T-Domäne für die Aktivität der E-Domänen

Dieses Beispiel dient auch als Beispiel für Modifikationsgeneratoren (s. Beispiel 3). Es wurden künstliche Fusionsproteine von A-Domänen mit TE-Didomänen (Typ A-TE) sowie von AT-Didomänen mit E-Domänen (Typ AT-E) hergestellt.

Dabei stellte sich die Wichtigkeit der richtig gewählten T-Domäne heraus, um aktive Proteine zu erhalten. Die Konstrukte des Typs AT_{C}-E waren in Bezug auf die Epimerisierungsreaktion inaktiv, die des Typs AT_{E}-E bzw. A-T_{E}E dagegen aktiv ("-" entspricht der Fusionsstelle).

Die jeweiligen DNA-Fragmente wurden mit geeigneten Oligonukleotiden durch PCR aus chromosomaler DNA amplifiziert und zunächst in geeignete Vektoren zwischenkloniert. In einem zweiten Klonierungsschritt wurden die Vektoren mit den entsprechenden Restriktionsenzymen hydrolytisch gespalten und die aufgereinigten DNA-Fragmente in gewünschter Art und Weise ligiert.

Die verwendeten Fragmente (in Klammern dahinter die zur Amplifikation verwendeten Primer) waren TE(tycA) (Seq ID-NO:47 und Seq ID NO:48; Größe des Amplifikates 1710 bp), E(tycA) (Seq ID-NO:49 und Seq ID-NO:48; 1476 bp), A(tycB2) (Seq ID-NO:50 und Seq ID-NO:51; 1570 bp), AT(tycB2) (Seq ID-NO:50 und Seq ID-NO:52; 1804 bp), A(tycB3) (Seq ID-NO:53 und Seq ID-NO:54; 1559 bp), AT(tycB3) (Seq ID-NO:53 und Seq ID-NO:55; 1793 bp), A(tycC4) (Seq ID-NO:56 und Seq ID-NO:57; 1570 bp), A(bacA1) (Seq ID-NO:58 und Seq ID-NO:59; 1613 bp) und AT(bacA1) (Seq ID-NO:58 und Seq ID-NO:60; 1847 bp). Sämtliche tyc-Fragmente wurden aus chromosomaler DNA von *B*. *brevis* ATCC8185 und die beiden bac-Fragmente aus chromosomaler DNA von *B. licheniformis* ATCC 10716 amplifiziert und mit dem "QIA quick spin PCR purification kit" gereinigt.

Alle im nachfolgenden nach hydrolytische Spaltung mit Restriktionsendonukleasen erhaltenen DNA-Fragmente wurden mit dem "QIA quick spin PCR pruification kit" gereinigt. Die beiden E-Domänen-Fragmente TE(tycA) und E(tycA) wurden mit den Restriktionsendonukleasen Bam HI und Bgl II hydrolytisch gespalten, gereinigt und jeweils in Bglll-hydrolytisch gespaltenen-pQE60-Vektoren mittels T4 DNA Ligase ligiert. Die übrigen Fragmente wurden in pQE-60 bzw. pQE70-Vektoren über die Restriktionsschnittstellen Ncol/BamHI (pQE60) bzw. Sphl/BamHl (pQE70) kloniert. Dabei erfolgte zunächst die hydrolytische Spaltung der Amplifikate mit den angegebenen Restriktionsendonukleasen, die Reinigung der DNA-Fragmente und die Ligierung mittels T4 DNA Ligase in die gleichermaßen behandelten, angegeben Vektoren. Ein Aliquot der Ligationsansätze wurde jeweils zur Transformation von E. coli XL1Blue eingesetzt, Transformanten auf LB-Agar Platten (Ampicillin 100 µg/mL) selektiert und zur Plasmidpräparation verwendet. Korrekte Vektorkonstruktion wurden durch Restriktionsanalysen und terminale DNA Sequenzierung identifiziert. So wurden die Vektoren pQE60-E(tycA), pQE60-TE(tycA), pQE70-A(tycB2), pQE70-AT(tycB2), pQE70-A(tycB3), pQE70-AT(tycB3), pQE60-A(tycC4), pQE60-A(bacA1) und pQE60-AT(bacA1) erhalten.

Die DNA-Fragmente von E(tycA) bzw. TE(tycA) konnten nun aus den Vektoren pQE60-E(tycA) bzw. pQE60-TE(tycA) durch hydrolytische Spaltung mit Bgl II und Nde I zusammen mit einem Teil des pQE-Vektors isoliert werden. Die Plasmide, die die für die A-Domänen bzw. AT-Didomänen kodierenden Fragmente trugen wurden mit Bam HI und Nde 1 hydrolytisch gespalten und nach Reinigung mit T4 DNA Ligase mit den entsprechenden für E(tycA) bzw. TE(tycA) kodierenden Fragmenten ligiert. Nach jeweiliger Transformation eine Aliquots des Ligationsansatzes mit E. coli XL1 Blue, Selektion von Transformanten auf LB-Agar Platten (Ampicillin 100 µg/mL), und Plasmidpräparation aus den erhaltenen Kolonien konnten so die Vektorkonstrukte für die künstlichen Hybridproteine gewonnen werden: pQE70-A(tycB2)-TE(tycA), pQE70-AT(tycB2)-E(tycA), pQE70-A(tycB3)-TE(tycA), pQE70-AT(tycB3)-E(tycA), pQE60-A(tycC4)-TE(tycA), pQE60-A(bacA1)-TE(tycA) und pQE60-AT(bacA1)-E(tycA). Diese Expressionvektoren wurden zur Transformation von E. coli M15/pREP4 eingesetzt und Transformanten auf LB-Agar Platten (Ampicillin 100 µg/mL; Kanamycin 25 µg/mL) selektiert. Die so erhaltenen Stämme wurden nun zur Proteinproduktion wie in Beispiel 1 c beschrieben eingesetzt. Die Proteinreinigung erfolgte ebenfalls analog der in Beispiel 1 c beschriebenen Methode. Nach der Dialyse gegen Assaypuffer (100 mM NaCl, 50 mM HEPES, 1 mM EDTA, pH 8,0) wurde die Proteinlösung aliquotiert, mit flüssigem Stickstoff schockgefroren und bei -80°C gelagert. Für die Enzymreaktionen wurde je ein neues Aliquot auf Eis aufgetaut. Die erhaltenen Proteine hiessen: A(tycB2)-TE(tycA), AT(tycB2)-E(tycA), A(tycB3)-TE(tycA), AT(tycB3)-E(tycA), A(tycC4)-TE(tycA), A(bacA1)-TE(tycA) und AT(bacA1)-E(tycA) ("-" entspricht der Fusionsstelle).

Für die Enzymreaktionen wurden die Enzyme (je 100 pmol) in Assaypuffer (100 µL Gesamtvolumen) mit Sfp (5 pmol, aus *B*. *subtilis),* MgCl₂ (10 mM) und CoA (0,05 mM) 10 min bei 37°C präinkubiert und so posttranslational mit dem Cofaktor 4'-Phosphopanthetein modifiziert. Nach Zugabe der jeweiligen ¹⁴C-markierten L-Aminosäure ([¹⁴C]-L-Phe für A(tycB2)-TE(tycA), AT(tycB2)-E(tycA), A(tycB3)-TE(tycA) und AT(tycB3)-E(tycA), [¹⁴C]-L-Val für A(tycC4)-TE(tycA), [¹⁴C]-L-Ile für A(bacA1)-TE(tycA) und AT(bacA1)-E(tycA)) und ATP (5 mM) wurde wiederum 10 min bei 37°C inkubiert. Die Präzipitation der Enzyme erfolgte mit 1 ml 10% TCA-Lsg. Nach 15 min Inkubation auf Eis wurde 25 min bei 13000 Upm bei 4°C zentrifugiert und der Überstand entfernt. Nach zweimaligem Waschen des Pellets mit 10% TCA (0,8 mL), je einmaligem Waschen mit Diethylether/Ethanol (3:1) und Diethylether (jeweils 1 mL) wurde bei 37°C im Heizblock getrocknet. Nach Zugabe von 100 µl 100 mM KOH wurde 10 min bei 75°C im Schüttelinkubator (14000 Upm) inkubiert. Nach der Zugabe von 1 mL Methanol wurde 30 min bei 13000 Upm/4°C zentrifugiert und der Überstand in ein neues Reaktionsgefäß überführt. Nach dem Entfernen des Überstandes unter Vakuum wurde das Pellet in 20 µl 50% Ethanol resuspendiert und je 10 µl auf HPTLC-Fertigplatten CHIR (Merck, Darmstadt) aufgetragen. Die Entwicklung der Dünnschichtchromatographien erfolgte mit Acetonitril/Methanol/Wasser (4:1:1). Der Nachweis der Substanzen erfolgte durch Autoradiographie.

Eine Epimerisierungsaktivitität konnte nach der Reaktion durch ein Gemisch aus den D- und L-Aminosäuren nachgewiesen werden (R_{f}-Werte: L-Phe 0.6; D-Phe 0.47; L-lle 0.55; D-lle 0.48; L-Val 0.52; D-Val 0.42). Die beiden Enzyme AT(tycB2)-E(tycA) und AT(bacA1)-E(tycA) mit einer T-Domäne, die natürlicherweise N-terminal vor einer C-Domäne liegt, also einer T_{C}-Domäne, zeigten keine Epimerisierungsaktivität (Typ AT_{C}-E), während im Falle von AT(tycB3)-E(tycA) eine Epimerisierungsreaktion zu beobachten war (Typ AT_{E-}E). Im letzteren Fall liegt die T-Domäne auch natürlicherweise vor einer E-Domäne, es handelt sich also um eine sogenannte T_{E}-Domäne. Durch Fusion vor der T_{C}-Domäne, also Verbindung der A-Domäne mit der T_{E}-Domäne, auf die eine E-Domäne folgt, gelang aber auch die Epimerisierung der aktivierten Aminosäure durch die Hybridenzyme A(tycB2)-TE(tycA), A(tycB3)-TE(tycA), A(bacA1)-TE(tycA) und A(tycC4)-TE(tycA) (Typ A-T_{E}E).

### Beispiel 2a: Konstruktion von Hybridpeptidsynthetasen für die Produktion eines bekannten Peptidantibiotikums:

Dieses Beispiel erläutert die Konstruktion von drei Peptidsynthetasen Pa, Pb und Pc nach dem erfindungsgemäßen Verfahren aus Genfragmenten verschiedener Peptidsynthetasengene, die zusammen die Synthese des Peptidgerüsts des Lipopeptidantibiotikums Plipastatin (Tsuge et al. (1996), Arch. Microbiol. 165:243-251) katalysieren. Das vollständige Plipastatin erhält man, wenn man zusätzlich zu allen Substraten (ATP, MgCl₂ und Aminosäuren, s.u.) einen Zellextrakt aus *Bacillus subtilis* JH642 einsetzt, der als Donor der β-hydroxy-Fettsäure erforderlich ist. Wird dagegen die modifizierte Peptidsynthetase Pa2 mit Pb und Pc inkubiert, wird stattdessen eine fettsäurefreie Variante von Plipastatin hergestellt.

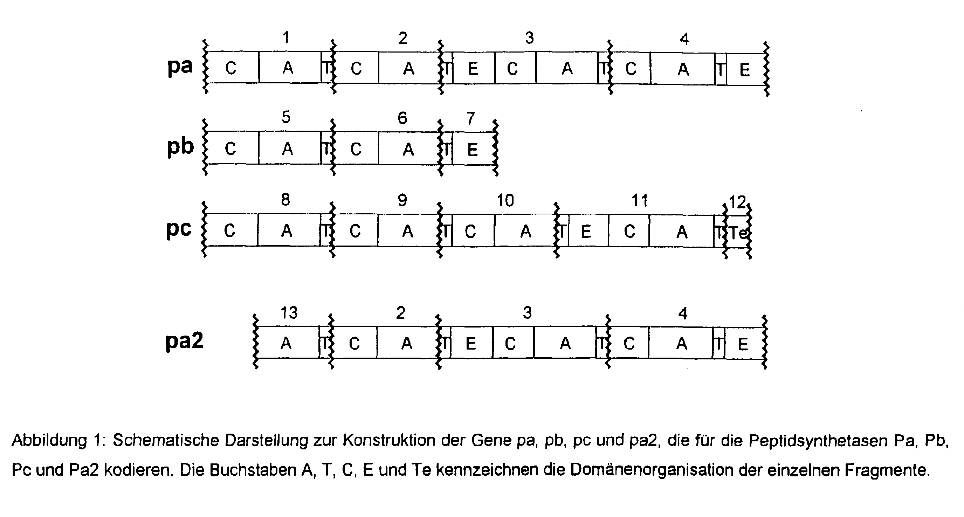

Eine schematische Darstellung des Aufbaus der Gene pa, pb, pc und pa2, die für die Peptidsynthetasen Pa, Pb, Pc und Pa2 kodieren wird in Figur 1 gegeben. Die Buchstaben A, T, C, E und Te kennzeichnen dabei die Domänenorganisation der einzelnen Fragmente. Die Numerierung der Fragmente stimmt mit der in der weiter unten stehenden Tabelle 1 verwendeten Numerierung überein.

Für die Konstruktion der rekombinanten Peptidsynthetasen zur Produktion des Antibiotikums Plipastatin unter Verwendung von Fragmenten aus anderen für Peptidsynthetasen kodierenden Genen werden schrittweise 3 Plasmide (pPa, pPb und pPc) hergestellt. Dabei wird von dem Vektor pTZ18R (Pharmacia, 41 Deutschland) ausgehend in einem Zyklus 1) mit Xba I (Amersham/Buchler, Braunschweig, Deutschland, Best.-Nr.: E1093Y) hydrolytisch gespalten, 2) mit CIP Phosphatase dephosphoryliert und das gereinigte DNA-Fragment mit 3) dem jeweils nächsten PCR-Fragment, welches zuvor mit Xba I und Nhe I (Amersham/Buchler, Braunschweig, Deutschland, Best.-Nr.: E1162Y) hydrolytisch gespalten wurde, mittels T4 DNA Ligase ligiert. 4) Kompetente Escherichia coli-Zellen werden mit einem Aliquot des Ligationsansatzes transformiert und von 5) nach Selektion auf Ampicillin erhaltenen Kolonien 6) extrachromosomale DNA präpariert. 7) Die gewünschte Plasmidkonstruktion, d.h. die Insertion des DNA-Fragmentes in das Plasmid in richtiger Orientierung, wird durch geeignete Restriktionsanalysen ermittelt. Ein so erhaltenes Plasmid kann für die Insertion des nächsten DNA-Fragmentes verwendet werden.

**Tabelle 1: Oligonukleotide und Herkunft der chromosomalen DNA für die PCR-Amplifikation der erforderlichen DNA-Fragmente**

| Fragment Nr. | Oligonukleotide Seq ID-NO: | Aus chromosomaler DNA von Stamm |
|---|---|---|
| 1 | 10&11 | *Bacillus subtilis* JH642 |
| 2 | 13 & 14 | *Bacillus brevis* ATCC8185 |
| 3 | 15 & 16 | *Amycolatopsis orientalis* |
| 4 | 17 & 18 | *Bacillus subtilis* JH642 |
| 5 | 19 & 20 | *Bacillus subtilis* J H642 |
| 6 | 21 & 22 | *Bacillus brevis* ATCC8185 |
| 7 | 23 & 24 | *Bacillus brevis* ATCC8185 |
| 8 | 25 & 26 | *Bacillus brevis* ATCC8185 |
| 9 | 27 & 28 | *Bacillus licheniformis* ATCC10716 |
| 10 | 29 & 30 | *Bacillus brevis* ATCC8185 |
| 11 | 31 & 32 | *Bacillus licheniformis* ATCC10716 |
| 12 | 33 & 34 | *Bacillus subtilis* JH642 |
| 13 | 12 & 11 | *Bacillus subtilis* JH642 |

Für pPa wird die beschriebene Prozedur schrittweise mit den DNA-Fragmenten 1, 2, 3, 4 und 5 durchgeführt. Für pPa2 wird die beschriebene Prozedur schrittweise mit den DNA-Fragmenten 13, 2, 3, 4 und 5 durchgeführt. Für pPb wird die beschriebene Prozedur schrittweise mit den DNA-Fragmenten 5, 6 und 7 durchgeführt. Für pPc wird die beschriebene Prozedur schrittweise mit den DNA-Fragmenten 8, 9, 10 11 und 12 durchgeführt. Siehe Tabelle 1 für die jeweils zu verwendenden Oligonukleotide und chromosomale DNA. Siehe Abbildung 1 für eine schematische Darstellung der so konstruierten rekombinanten NRPS-Gene.

Die Plasmide pPa, pPa2, pPb und pPc werden anschließend jeweils in einen geeigneten *E*. *coli* Expressionsstamm transformiert, z.B. *E. coli* BL21/pREP4-gsp (Stachelhaus et al. (1998), J. Biol. Chem. 273: S.22773-22781). Dieser Stamm ermöglicht die Koexpression mit dem Gen gsp, das für eine 4'-Phosphopantethein-Transferase kodiert, welche die posttranslationale Modifikation von Peptidsynthetasen mit dem Kofaktor 4'-Phoshopantethein katalysiert. Der Vektor pREP4-gsp verleiht Resistenz gegen Kanamycin, so daß Transformanten mit Kanamycin (25 µg/mL) und Ampicillin (100 µg/mL) selektiert werden. Die so erhaltenen Stämme BL21/pREP4-gsp/pPa, BL21/pREP4-gsp/pPb und BL21/pREP4-gsp/pPc werden wie beschrieben (Stachelhaus et al. (1998), J. Biol. Chem. 273: S.22773-22781) in Flüssigmedium zur Proteinproduktion eingesetzt. Die Reinigung der rekombinanten Proteine Pa, Pa2, Pb und Pc kann durch übliche dem Fachmann bekannte Techniken erfolgen.

Eine andere Möglichkeit um mit 4'-Phosphopantethein modifizierte Peptidsynthetasen zu erhalten, ist die in vitro Modifikation durch Zugabe der 4'-Phoshopantethein-Transferase Sfp, Coenzyme A und MgCl₂ (Lambalot et al. (1996) Chem. & Biol. 3: S.923-936).

Die gereinigten Proteine Pa, Pb und Pc werden in einem geeigneten Puffer (z.B. HEPES 50 mM, 100 mM NaCl, pH 8,0) in äquimolaren Mengen (z. B. je 500 nM) mit ATP (10 mM), MgCl₂ (10 mM) und allen Substrataminosäuren (Glutamat, Ornithin, Tyrosin, allo-Threonin, Valin, Prolin und Isoleucin, alle 1 mM) bei 37°C inkubiert. Durch Zugabe einer Fraktion, die durch Gelfiltration eines Rohzellextraktes von *Bacillus subtilis* ATCC 21332 erhalten wird und Proteine des Molekulargewichtes von ca. 40 kDa enthält (Menkhaus et al. (1993) J. Biol. Chem. 268: S.7678-7684), kann die Initiation der Synthese des Lipopeptids Plipastatin bewirkt werden.

Ein selbstinitiierendes System wird erhalten, indem die gereinigten Proteine Pa2, Pb und Pc in äquimolaren Mengen mit allen Substraten inkubiert werden. Diese drei Proteine synthetisieren die fettsäurefreie Variante des Plipastatin.

### Beispiel 2b:

Alternativ zur in vitro Synthese von Plipastatin nach dem in Beispiel 2a beschriebenen Verfahren mit den konstruierten Peptidsynthetasen Pa, Pb und Pc, kann eine chromosomale Integration mittels homologer Rekombination der für Pa, Pb und Pc kodierenden Fragmente in einen geeigneten Mikroorganismus erfolgen, um das Antibiotikum durch den so konstruierten Stamm zu synthetisieren. Als geeigneter Wirtsstamm kann insbesondere *Bacillus subtilis* ATCC21332 verwendet werden. Da die zu integrierenden Genfragmente teilweise aus den für die Surfactin und putativ aus den für die Fengycin Peptidsynthetasen kodierenden Genfragmenten bestehen, müssen diese in *Bacillus subtilis* ATCC 21332 für eine kontrollierte Integration zunächst deletiert werden. Dies kann jeweils durch dem Fachmann bekannte Methoden durchgeführt werden, indem 5'- und 3'-flankierende Bereiche des srfAA-(Surfactin Biosynthese) bzw. pps- (oder fen-, Fengycin Biosynthese) Operons in ein Plasmid kloniert werden und zwischen diese Fragmente eine übliche Antibiotikumsresistenzkassette, die Resistenz gegen z.B. Erythromicin, Spectinomycin, Kanamycin oder Chloramphenicol verleiht, kloniert wird (vergl. für diese Methode z. B. Schneider et al. (1998) Mol. Gen. Genet. 257: S.308-318). Dabei ist zu beachten, daß innerhalb des srfAA-Biosyntheseoperons das Gen comS lokalisiert ist, welches für die Kompetenzentwicklung von *Bacillus subtilis* essentiell ist (Schneider et al. (1998) Mol. Gen. Genet. 257: S.308-318). Dieses Gen muß daher in einem ersten Schritt in einer weiteren Kopie in das Chromosom von *Bacillus subtilis* integriert werden, z. B. in das amyE-Gen. Die Integration der für Pa, Pb und Pc kodierenden Genfragmente erfolgt idealerweise in drei Schritten, z. B. an den ursprünglichen Lokalisationen des srfAA- oder pps- (oder fen-) Operons unter Verwendung der jeweiligen Promotoren. Für eine stabile Integration mittels Doppelcrossover müssen dazu stets 5'- und 3'- homologe Sequenzen zwischen den Plasmid-kodierten Genfragmenten für Pa, Pb und Pc, sowie dem Chromosom von *Bacillus subtilis* vorhanden sein. Eine positive Selektion auf Integration des Genfragmentes kann durch Verwendung von üblichen Genkassetten erfolgen, welche Resistenzen gegen Antibiotika wie z.B. Erythromycin, Spectinomycin, Kanamycin oder Chloramphenicol verleihen.

Eine chromosomale Integration, z. B. in *Bacillus subtilis* ATCC 21332, der für Pa, Pb und Pc kodierenden Genfragmente in Operonorganisation unter der Kontrolle eines geeigneten Promotors, z. B. des srfAA-Promotors, führt zur in vivo-Produktion der drei Peptidsynthetasen Pa, Pb und Pc, welche von dem *Bacillus subtilis* ATCC 21332-Protein Sfp mit dem Kofaktor 4'-Phosphopantethein modifiziert werden und dann die Synthese von Plipastatin bewerkstelligen.

### Beispiel 2c: Konstruktion eines NRPS-PKS-Hybridsystems

In diesem Beispiel wird das Prolin-einbauende Modul ProCAT (siehe Beispiel 1 a) mit dem Propionat-einbauenden Modul 6 der 6-Deoxyerythronolide B Synthase (DEBS1-3, kodiert durch die Gene eryAl, eryAII und eryAIII, DEBS3 enthält die Module 5 und 6 (Staunton et al. (1997), Chem. Rev. 97, S.2611-2629)) fusioniert . Die Fusionsstelle liegt dabei nach dem erfindungsgemäßen Verfahren bei Aminosäure 38 und 39 nach dem konservierten Serin der T-Domäne von ProCAT. Das Modul DEBS3_6 wird gleichermaßen generiert, indem die Fusionsstelle Aminosäure 38 und 39 nach der ACP-Domäne des darauffolgenden Moduls 5 gewählt wird. Das Modul DEBS3_6 enthält eine terminale Te-Domäne, die zur Produktabspaltung dient.

Für die Konstruktion eines Expressionsplasmides pProCAT-DEBS3_6 wird ausgehend von pQE60-ProCAT (siehe Beispiel 1a) verfahren. Aus chromosomaler DNA von Saccharopolyspora erythraea wird per PCR mit den Oligonukleotiden Seq ID-NO:45 und Seq ID-NO:46 ein Fragment von 5136 bp Größe amplifiziert. Das Amplifikat wird mit dem "Qia Quick spin purification kit" gereinigt und anschließend mit den Restriktionsendonukleasen BglII und Bam HI terminal behandelt. Nach erneuter Aufreinigung wird dieses Fragment mit T4-DNA Ligase mit dem Plasmid pQE60-ProCAT ligiert, das zuvor mit Bam HI hydrolytisch gespalten, mit alkalischer Phosphatase dephosphoryliert und gereinigt wurde. Ein Aliquot des Ligationsansatzes wird zur Transformation von E.coli XL1 Blue eingesetzt. Transformanten werden auf LB-Agar Platten (100 µg/mL Ampicillin) selektiert. Ampicillin-resistente Kolonien werden zur Plasmidpräparation eingesetzt. Gewünschte Plasmidkonstruktionen werden durch Restriktionsanalysen mit Resitrktionsendonukleasen identifiziert. So hergestellter Expressionvektor, pProCAT-DEBS3_6 ("-" entspricht der Fusionsstelle), wird zur Transformation von E. coli BL21-pREP4-gsp eingesetzt. Transformanten werden auf LB-Agar Platten (100 µg/mL Ampicillin; 25 µg/mL Kanamycin) selektiert und weiterhin zur Produktion des rekombinaten Proteins ProCAT-DEBS3_6 eingesetzt. Produktion und Reinigung von ProCAT-DEBS3_6 erfolgen dabei analog den Beispielen 1a-c.

Zur Produktbildung inkubiert man 50 pmol des Enzyms ProCAT-DEBS3_6 mit 50 pmol TycA (siehe Beispiel 1a) in einem geeigneten Puffer in einem Gesamtvolumen von 100 µL. Im Reaktionsmix sind ebenfalls enthalten: 1 mM L-Phenylalanine, 1 mM L-Prolin, 0,1 mM (DL) Methylmalonyl-CoA, 0,1 mM NADPH, 10 mM MgCl₂ und 5 mM ATP. Der Reaktionsmix wird für 2 h bei 30°C inkubiert, durch Zugabe von 50 µL Butanol abgestoppt und unter vermindertem Druck bis zur Trockne eingeengt. Das Pellet wird in 10 % Methanol aufgenommen und so zur Produktanalyse mittles HPLC/MS eingesetzt. Das Enzymsystem TycA plus ProCAT-DEBS3_6 synthetisiert das Produkt 3-{(2S)-1-[(2*R*)-2-Amino-3-phenylpropanoyl]tetrahydro-1*H*-2-pyrrolyl}-3-hydroxy-2-methylpropansäure.

### Beispiel 3: Modifikationsgenerator

Das erfindungsgemäße Verfahren ermöglicht auch die Konstruktion einer monomodularen Peptidsynthetase, die eine spezifische L-Aminosäure erkennt, aktiviert, in die D-Konfiguration umwandelt und schließlich als freie D-Aminosäure abspaltet.

Dazu wird ein für A-, T- und E- Domänen kodierendes Peptidsynthetasengenfragment mit dem für eine Te-Domäne kodierenden Fragment fusioniert. Das in Beispiel 1a beschriebene Plasmid pTycA mit Bam HI wird hydrolytisch gespalten, die entstandenen freien DNA-Enden werden dephosphoryliert (mit CIP-Phosphatase), und dieses DNA-Fragment wird mit dem PCR-Amplifikat für die Te-Domäne (s. Beispiel 1 b) ligiert, welches zuvor wie oben beschrieben mit Bam HI und Bgl II hydrolytisch gespalten wird. Das so erhaltene Plasmid pTycA-Te ("-" entspricht der Fusionsstelle) wird eingesetzt wie in Beispiel 1 a beschrieben zur Expression der Peptidsynthetase TycA-Te, die freies L-Phenylalanin erkennt, aktiviert, racemisiert und schließlich als D-Phenylalanin abspaltet.

Alternativ zum für die Te-Domäne kodierenden Fragment der Tyrocidin-Biosynthesegene können andere für Te-Domänen kodierende Fragmente verwendet werden, insbesondere solche die in den Peptidsynthetasen, aus deren Genen sie gewonnen werden, auf eine E-Domäne folgen (z.B. ACV-Synthetasen).

Für Umwandlung anderer Aminosäuren von der L- in die D-Form können nach dem erfindungsgemäßen Verfahren A-Domänen anderer Aminosäurespezifität mit einer T-, E- und Te-Domäne verwendet werden.

**Tabelle 2: Für die Beispiele 1-3 verwendete Oligonukleotide (alle von MWGBiotech, Ebersberg, Deutschland)**

| Seq ID-NO: | Sequenz Oligonukleotid 5'-...-3' |
|---|---|
| 1 | ATACCATGGT AGCAAATCAG GCCAATC |
| 2 | TATGGATCCG CGCAGTCTAT TTGCAAG |
| 3 | TATCCATGGG TGTATTTAGC AAAGAACAAG TTC |
| 4 | TATGGATCCT TCCACATACG CTGCCAG |
| 5 | ATAGGATCCG CCAAAGGGAA TGTCTTCTCG |
| 6 | ATAGGATCCT TTCAGGATGA ACAGTTCTTG |
| 7 | ATAGGATCCG CATTCGAGCA GTTCGAG |
| 8 | ATAGGATCCT TCGATGAACG CCGCCAG |
| 9 | ATAAGATCTC ATAAGCGCTT TGAGAGCAG |
| 10 | ATAGCTAGCG GAGGATCACA TGGAAATAAC TTTTTACCCT |
| 11 | ATATCTAGAA TCAGCTTCCT CTGCAAG |
| 12 | ATAGCTAGCG AGAAAGAGAA GCTGCTTG |
| 13 | ATAGCTAGCG CATTCGAGCA GTTCGAG |
| 14 | ATATCTAGAT CTCAGCATGG TGACATC |
| 15 | ATAGCTAGCC GCGAACCGCG CACCGA |
| 16 | ATATCTAGAT TTGACCATGG CCGCCAG |
| 17 | ATAGCTAGCA AAGAACACGC TTTTACACAG |
| 18 | ATATCTAGAT CCTCTCCTAT AGTTTGTTG |
| 19 | ATAGCTAGCG GAGATTCACT GATGCCG |
| 20 | ATATCTAGAA CGGATAACGG TAGCCAG |
| 21 | ATAGCTAGCG GAAAAGAGAC GTATGTGC |
| 22 | ATATCTAGAT GTCGTCCGCT CGCCGG |
| 23 | ATAGCTAGCG CTGCCTACCA TCCTCC |
| 24 | ATATCTAGAC AACATCTGTT TAGCGCAG |
| 25 | ATAGCTAGCG GAGGTGCCGT AATGAG |
| 26 | ATATCTAGAT TCCACATACG CTGCCAG |
| 27 | ATAGCTAGCC GAAGGAAATG CTTTTACATC |
| 28 | ATATCTAGAG ACATTTACAT TGCCGTCG |
| 29 | ATAGCTAGCG TGTATGTGGC GCCGCG |
| 30 | ATATCTAGAG GTTACGGGCT TGCCTTC |
| 31 | ATAGCTAGCG GAACCGGGTA CGATCC |
| 32 | ATATCTAGAT AATACAAAAC GCGCTAATG |
| 33 | ATAGCTAGCA AACAGCTGAC AGCAGCAA |
| 34 | ATATCTAGAT CCGCTTTATC GTTTGTGC |
| 35 | TTTCCATGGC TAAACATTCA TTAGA |
| 36 | TTCCTGCAGC GCCCCCGCCG TTCTG |
| 37 | AGCCTGCAGG CCTACCATCC TCCGAG |
| 38 | TGGACCCATG GTAATTTCTC CTCT |
| 39 | ATACTGCAGG AGTATGTAGC GCCGC |
| 40 | TATGGATCCT TTCAGGATGA ACAGTTCTTG |
| 41 | ACCGTTAACG AATACGTGGC CCCGAG |
| 42 | AATGTTAACC TCCTGCAGCG CCCC |
| 43 | ACGCTGCAGG ATTACGTCGC CCCGA |
| 44 | AGCGTTAACT GTTGCAGGCT TTCCTTC |
| 45 | TATCCATGGG AAGATCTCTC GTCGGCGCAG CAGAG |
| 46 | ATAGGATCCT GAATTCCCTC CGCCCA |
| 47 | TAAAGATCTG CCTACCATCC TCCG |
| 48 | TATGGATCCG CGCAGTGTAT TTGCAAG |
| 49 | ATAAGATCTA GAAAAAGCGA TCAGGGCATC |
| 50 | AATGCATGCT GACTGCGCAT GAG |
| 51 | TAAGGATCCT GTTGCAGGCT TTCC |
| 52 | ATAGGATCCT TCGATCAAGC GGGCCAAGTC |
| 53 | AAAGCATGCT GACAGCAGCA G |
| 54 | AAAGGATCCC CGGTTCTCCT CCTGGTT |
| 55 | AAAGGATCCC GGGATGACGC GCAGAG |
| 56 | AATCCATGGT CAGCGAGGAA GAGCG |
| 57 | AAAGGATCCT GTCGTCCGCT CG |
| 58 | AAACCATGGT TGCTAAACAT TCATTAG |
| 59 | AAAGGATCCC GCCCCGCCGT TCTG |
| 60 | AAAGGATCCT TCGATAAAAG CGCTC |

## Patentansprüche

1. Maßgeschneiderte artifizielle nicht-ribosomale Peptidsynthetase (NRPS) zur nicht-ribosomalen Synthese von Peptiden einer vorbestimmten Länge und Zusammensetzung und/oder zur nicht-ribosomalen Modifikation von Peptiden oder Aminosäuren, wobei die NRPS mehrere Domänen umfasst, deren Abfolge die Struktur des Peptids bestimmt und/oder die Modifikation des Peptids oder der Aminosäure beinflusst, wobei eine Adenylierungsdomäne (A-Domäne) und eine Thiolierungsdomäne (T-Domäne), gegebenenfalls eine Kondensationsdomäne (C-Domäne) oder eine Cyclisierungsdomäne (Cy-Domäne), gegebenenfalls eine Thioesterasedomäne (Te-Domäne) oder eine Reduktasedomäne (R-Domäne) sowie gegebenenfalls mindestens eine Modifikationsdomäne ein Modul darstellen, wobei jedes Modul für eine vorbestimmte Aminosäure charakteristisch ist, und wobei jedes Modul zwischen mindestens einer der Domänen und/oder zwischen aufeinanderfolgenden Modulen Linkerbereiche aufweist, **dadurch gekennzeichnet, daß** die NRPS durch gezielte Fusion von Domänen oder von Abfolgen von Domänen und/oder von Modulen oder von Abfolgen von Modulen in einem oder mehreren vorbestimmten Linkerbereichen erhältlich ist und dass die Fusion in einem oder mehreren der Linkerbereiche erfolgt ist:
a) im Linkerbereich zwischen der T- und der folgenden Domäne zwischen den Aminosäuren 34 bis 45 (jeweils einschließlich) carboxyterminal des 4'-Phosphopantethein-bindenden Serins der Sequenz DxFFxxLGG(DH)S(IL) der T-Domäne, und/oder
b) im Linkerbereich zwischen einer A- und einer T-Domäne im Bereich Aminosäure 10-18 (jeweils einschließlich) carboxyterminal zu dem Lysin der Sequenz NGK(VL)DR (Sequenz A10 der A-Domäne), und/oder
c) im Linkerbereich zwischen einer C- und einer A-Domäne im Bereich Aminosäure 38-60 (jeweils einschließlich) aminoterminal zu dem Leucin der Sequenz L(TS)YxEL (Sequenz A1 der A-Domäne), und/oder
d) im Linkerbereich zwischen einer E- und einer C-Domäne im Bereich Aminosäure 9-28 (jeweils einschließlich) aminoterminal zu dem Serin der Sequenz SxAQxR(LM)(WY)xL (Sequenz C1 der C-Domäne), wobei die Sequenzbezeichnungen nach Marahiel et al. (1997), Chem. Rev. 97: S.2651-2673, erfolgen.

2. Maßgeschneiderte artifizielle nicht-ribosomale Peptidsynthetase nach Anspruch 1, **dadurch gekennzeichnet, daß** die gezielte Fusion in einem Linkerbereich zwischen einer T-Domäne und der folgenden Domäne, in einem Linkerbereich zwischen einer A- und einer T-Domäne, in einem Linkerbereich zwischen einer C- und einer A-Domäne und/oder in einem Linkerbereich zwischen einer E- und einer C-Domäne erfolgt ist.

3. Maßgeschneiderte artifizielle nicht-ribosomale Peptidsynthetase-Polyketidsynthase-Hybridsysteme (NRPS-PKS) nach Anspruch 1, **dadurch gekennzeichnet, daß** diese Domänen, Abfolgen von Domänen, Module oder Abfolgen von Modulen enthält, die sich von Polyketidsynthasen (PKS) ableiten, wobei eine Acyltransferase (AT), ein Acyl-Carrier-Protein (ACP), gegebenenfalls eine Ketosynthase (KS), und gegebenenfalls reduktive Domänen ein Modul darstellen, wobei jedes Modul für den Coenzym-A-Ester der Malonsäure oder einer α-substituierten Malonsäure charakteristisch ist, und wobei jedes Modul zwischen mindestens einer der Domänen und/oder zwischen aufeinander-folgenden Modulen Linkerbereiche aufweist, und die NRPS-PKS durch gezielte Fusion von Domänen oder von Abfolgen von Domänen und/oder von Modulen oder von Abfolgen von Modulen in einem oder mehreren vorbestimmten Linkerbereichen erhältlich ist, wobei die Fusion in einem oder mehreren der Linkerbereiche erfolgt ist:
e) im Linkerbereich zwischen einer T- und einer darauffolgenden KS-Domäne zwischen den Aminosäuren 34 bis 45 (jeweils einschließlich) carboxyterminal des Serins der Sequenz DxFFxxLGG(DH)S(IL) der T-Domäne, und/oder
f) im Linkerbereich zwischen einer ACP-Domäne und einer darauf folgenden Domäne zwischen den Aminosäuren 34 bis 45 (jeweils einschließlich) carboxyterminal des 4'-Phosphopantethein-bindenden Serins der Sequenz (LI)GxDSL der ACP-Domäne, und/oder
g) im Linkerbereich zwischen einer A- und einer ACP-Domäne zwischen den Aminosäuren 10-18 (jeweils einschließlich) carboxyterminal zu dem Lysin der Sequenz NGK(VL)DR der A-Domäne und zwischen den Aminosäuren 46-77 aminoterminal zu dem 4'-Phosphopantethein-bindenden Serin der Sequenz (LI)GxDSL der ACP-Domäne.

4. Maßgeschneiderte artifizielle nicht-ribosomale Peptidsynthetase nach Anspruch 1, **dadurch gekennzeichnet, daß** die Modifikationsdomäne ausgewählt wird aus der Gruppe bestehend aus der Epimerisierungsdomäne (E-Domäne), der N-Methylierungsdomäne (M-Domäne), der N-Formylierungsdomäne (F-Domäne) oder der Oxidations-domäne (Ox-Domäne).

5. Maßgeschneiderte artifizielle nicht-ribosomale Peptidsynthetase nach Anspruch 1, **dadurch gekennzeichnet, daß** diese mindestens ein Modul enthaltend eine A-, T- und C-Domäne und/oder mindestens ein Modul enthaltend eine A-, T- und Cy-Domäne aufweist.

6. Maßgeschneiderte artifizielle nicht-ribosomale Peptidsynthetase nach Anspruch 1, **dadurch gekennzeichnet, daß** diese mindestens einen Linkerbereich aufweist, der durch Fusion zwischen der T-Domäne und einer folgenden C-, E-, Cy-, R- oder Te-Domäne erhalten wurde.

7. Maßgeschneiderte artifizielle nicht-ribosomale Peptidsynthetase nach Anspruch 3, **dadurch gekennzeichnet, daß** diese mindestens einen Linkerbereich aufweist, der durch Fusion zwischen der T-Domäne und einer folgenden KS-Domäne erhalten wurde.

8. Maßgeschneiderte artifizielle nicht-ribosomale Peptidsynthetase nach Anspruch 1, **dadurch gekennzeichnet, daß** diese mindestens einen Linkerbereich aufweist, der durch Fusion zwischen der T-Domäne und der nachfolgenden Domäne zwischen den Aminosäuren 38 und 39 (jeweils einschließlich) carboxyterminal des 4'-Phosphopantethein-bindenden Serins der Sequenz DxFFxxLGG(DH)S(IL) der T-Domäne erhalten wurde.

9. Maßgeschneiderte artifizielle nicht-ribosomale Peptidsynthetase nach Anspruch 1, **dadurch gekennzeichnet, daß** diese mindestens einen Linkerbereich aufweist, der durch Fusion zwischen einer A- und einer T-Domäne im Bereich Aminosäure 16 und 17 (jeweils einschließlich) carboxyterminal zu dem Lysin der Sequenz NGK(VL)DR (Sequenz A10 der A-Domäne) erhalten wurde.

10. Maßgeschneiderte artifizielle nicht-ribosomale Peptidsynthetase nach Anspruch 1, **dadurch gekennzeichnet, daß** diese mindestens einen Linkerbereich aufweist, der durch Fusion zwischen einer C- und einer A-Domäne im Bereich Aminosäure 47 und 48 (jeweils einschließlich) aminoterminal zu dem Leucin der Sequenz L(TS)YxEL (Sequenz A1 der A-Domäne) erhalten wurde.

11. Maßgeschneiderte artifizielle nicht-ribosomale Peptidsynthetase nach Anspruch 1, **dadurch gekennzeichnet, daß** diese mindestens einen Linkerbereich aufweist, der durch Fusion im Linkerbereich zwischen einer E- und einer C-Domäne im Bereich Aminosäure 20 und 21 (jeweils einschließlich) aminoterminal zu dem Serin der Sequenz SxAQxR(LM)(WY)xL (Sequenz C1 der C-Domäne) erhalten wurde.

12. Maßgeschneiderte artifizielle nicht-ribosomale Peptidsynthetase nach Anspruch 1, **dadurch gekennzeichnet, daß** diese ein Modul zur Modifikation einer vorgegebenen Aminosäure darstellt, die die für die umzusetzende Aminosäure affinen A- und T-Domänen, die gewünschte Modifikations-domäne und gegebenenfalls eine Te-Domäne oder eine R-Domäne enthält.

13. Maßgeschneiderte artifizielle nicht-ribosomale Peptidsynthetase nach Anspruch 3, **dadurch gekennzeichnet, daß** diese mindestens einen Linkerbereich aufweist, der durch Fusion im Linkerbereich zwischen einer T- und einer darauffolgenden KS-Domäne zwischen den Aminosäuren 38 und 39 (jeweils einschließlich) carboxyterminal des Serins der Sequenz DxFFxxLGG(DH)S(IL) der T-Domäne erhalten wurde.

14. Maßgeschneiderte artifizielle nicht-ribosomale Peptidsynthetase nach Anspruch 3, **dadurch gekennzeichnet, daß** diese mindestens einen Linkerbereich aufweist, der durch Fusion im Linkerbereich zwischen einer ACP-Domäne und einer darauf folgenden Domäne zwischen den Aminosäuren 38 und 39 (jeweils einschließlich) carboxyterminal des 4'-Phosphopantethein-bindenden Serins der Sequenz (LI)GxDSL der ACP-Domäne erhalten wurde.

15. Maßgeschneiderte artifizielle nicht-ribosomale Peptidsynthetase nach Anspruch 3, **dadurch gekennzeichnet, daß** diese mindestens einen Linkerbereich aufweist, der durch Fusion im Linkerbereich zwischen einer A- und einer ACP-Domäne zwischen den Aminosäuren 16 und 17 (jeweils einschließlich) carboxyterminal zu dem Lysin der Sequenz NGK(VL)DR der A-Domäne und zwischen den Aminosäuren 46-77 aminoterminal zu dem 4'-Phosphopantethein-bindenden Serin der Sequenz (LI)GxDSL der ACP-Domäne erhalten wurde.

16. Verfahren zur maßgeschneiderten Synthese artifizieller nicht-ribosomaler Peptidsynthetasen (NRPS) nach Anspruch 1 umfassend die Maßnahmen:
i) Auswahl einer ersten DNA-Sequenz, die für eine Domäne, eine Abfolge von Domänen, ein Modul oder eine Abfolge von Modulen kodiert, das eine vorbestimmte Aminosäure oder eine vorbestimmte Abfolge von Aminosäuren des gewünschten Peptids anlagern und umsetzen kann,
ii) Auswahl einer zweiten DNA-Sequenz, die für eine Domäne, eine Abfolge von Domänen, ein Modul oder eine Abfolge von Modulen kodiert, das eine weitere vorbestimmte Aminosäure oder eine weitere vorbestimmte Abfolge von Aminosäuren des gewünschten Peptids anlagern und umsetzen kann,
iii) Verbinden des 3' Endes der ersten DNA-Sequenz mit dem 5' Ende der zweiten DNA-Sequenz,
iv) gegebenenfalls Wiederholung der Schritte ii) und iii) mit DNA-Sequenzen gewünschter Länge und Zusammensetzung, bis die Länge und Zusammensetzung entsprechend dem gewünschten Peptid erreicht worden ist, und
v) Expression der so erhaltenen DNA in das entsprechende Polypeptid nach an sich bekannten Verfahren,
**dadurch gekennzeichnet, daß** die Fusion der einzelnen Domänen, Abfolgen von Domänen, Module oder Abfolgen von Modulen in DNA-Abschnitten erfolgt, die definierten Linkerbereichen der NRPS entsprechen, wobei die definierten Linkerbereiche der NRPS ausgewählt werden aus der Gruppe bestehend aus:
a) dem Linkerbereich zwischen der T- und der folgenden Domäne zwischen den Aminosäuren 34 bis 45 (jeweils einschließlich) carboxyterminal des 4'-Phosphopantethein-bindenden Serins der Sequenz DxFFxxLGG(DH)S(IL) der T-Domäne, und/oder
b) dem Linkerbereich zwischen einer A- und einer T-Domäne im Bereich Aminosäure 10-18 (jeweils einschließlich) carboxyterminal zu dem Lysin der Sequenz NGK(VL)DR (Sequenz A10 der A-Domäne), und/oder
c) dem Linkerbereich zwischen einer C- und einer A-Domäne im Bereich Aminosäure 38-60 (jeweils einschließlich) aminoterminal zu dem Leucin der Sequenz L(TS)YxEL (Sequenz A1 der A-Domäne), und/oder
d) dem Linkerbereich zwischen einer E- und einer C-Domäne im Bereich Aminosäure 9-28 (jeweils einschließlich) aminoterminal zu dem Serin der Sequenz SxAQxR(LM)(WY)xL (Sequenz C1 der C-Domäne), wobei die Sequenzbezeichnungen nach Marahiel et al. (1997), Chem. Rev. 97: S.2651-2673, erfolgen.

17. Verfahren zur maßgeschneiderten Synthese artifizieller nicht-ribosomaler Peptidsynthetase-Polyketidsynthase-Hybridsystemen (NRPS-PKS) nach Anspruch 16 umfassend die zusätzlichen Maßnahmen:
vi) Auswahl einer DNA-Sequenz, die für eine Domäne, eine Abfolge von Domänen, ein Modul oder eine Abfolge von Modulen kodiert, das eine vorbestimmte Acetat- oder Propionateinheit oder eine vorbestimmte Abfolge von Acetat- und/oder Propionat-Einheiten in das gewünschte Produkt anlagern kann,
vii) Verbinden des 3' Endes einer DNA-Sequenz aus Schritten i), ii) oder der Wiederholung dieser Schritte mit dem 5' Ende der DNA-Sequenz aus Schritt vi), und
viii) gegebenenfalls Wiederholung von Schritten vi), i) und/oder ii) mit DNA-Sequenzen gewünschter Länge und Zusammensetzung, bis die Länge und Zusammensetzung entsprechend dem gewünschten Peptid-Hybridsystem enthaltend Acetat- und/oder Propionateinheiten erreicht worden ist, **dadurch gekennzeichnet, daß** die Fusion der einzelnen Domänen, Abfolgen von Domänen, Modulen oder Abfolgen von Modulen in DNA-Abschnitten erfolgt, die definierten Linkerbereichen der NRPS-PKS entsprechen, wobei die definierten Linkerbereiche der NRPS-PKS ausgewählt werden aus der Gruppe bestehend aus
e) dem Linkerbereich zwischen einer T- und einer darauffolgenden KS-Domäne zwischen den Aminosäuren 34 bis 45 (jeweils einschließlich) carboxyterminal des Serins der Sequenz DxFFxxLGG(DH)S(IL) der T-Domäne, und/oder
f) dem Linkerbereich zwischen einer ACP-Domäne und einer darauf folgenden Domäne zwischen den Aminosäuren 34 bis 45 (jeweils einschließlich) carboxyterminal des 4'-Phosphopantethein-bindenden Serins der Sequenz (LI)GxDSL der ACP-Domäne, und/oder
g) dem Linkerbereich zwischen einer A- und einer ACP-Domäne zwischen den Aminosäuren 10-18 (jeweils einschließlich) carboxyterminal zu dem Lysin der Sequenz NGK(VL)DR der A-Domäne und zwischen den Aminosäuren 46-77 aminoterminal zu dem 4'-Phosphopantethein-bindenden Serin der Sequenz (LI)GxDSL der ACP-Domäne.

18. Verfahren zur maßgeschneiderten Synthese artifizieller nicht-ribosomaler Peptidsynthetasen (NRPS) nach Anspruch 16, **dadurch gekennzeichnet, daß gekennzeichnet**, daß die Fusion der einzelnen Domänen, Abfolgen von Domänen, Module oder Abfolgen von Modulen in DNA-Abschnitten erfolgt, die definierten Linkerbereichen der NRPS im Linkerbereich zwischen der T- und der folgenden Domäne zwischen den Aminosäuren 38 und 39 (jeweils einschließlich) carboxyterminal des 4'-Phosphopantethein-bindenden Serins der Sequenz DxFFxxLGG(DH)S(IL) der T-Domäne entsprechen.

19. Verfahren zur maßgeschneiderten Synthese artifizieller nicht-ribosomaler Peptidsynthetasen (NRPS) nach Anspruch 16, **dadurch gekennzeichnet, daß** die Fusion der einzelnen Domänen, Abfolgen von Domänen, Module oder Abfolgen von Modulen in DNA-Abschnitten erfolgt, die definierten Linkerbereichen der NRPS im Linkerbereich zwischen einer A- und einer T-Domäne im Bereich Aminosäure 16 und 17 (jeweils einschließlich) carboxyterminal zu dem Lysin der Sequenz NGK(VL)DR (Sequenz A10 der A-Domäne) entsprechen.

20. Verfahren zur maßgeschneiderten Synthese artifizieller nicht-ribosomaler Peptidsynthetasen (NRPS) nach Anspruch 16, **dadurch gekennzeichnet, daß** die Fusion der einzelnen Domänen, Abfolgen von Domänen, Module oder Abfolgen von Modulen in DNA-Abschnitten erfolgt, die definierten Linkerbereichen der NRPS im Linkerbereich zwischen einer C- und einer A-Domäne im Bereich Aminosäure 47 und 48 (jeweils einschließlich) aminoterminal zu dem Leucin der Sequenz L(TS)YxEL (Sequenz A1 der A-Domäne) entsprechen.

21. Verfahren zur maßgeschneiderten Synthese artifizieller nicht-ribosomaler Peptidsynthetasen (NRPS) nach Anspruch 16, **dadurch gekennzeichnet, daß** die Fusion der einzelnen Domänen, Abfolgen von Domänen, Module oder Abfolgen von Modulen in DNA-Abschnitten erfolgt, die definierten Linkerbereichen der NRPS im Linkerbereich zwischen einer E- und einer C-Domäne im Bereich Aminosäure 20 und 21 (jeweils einschließlich) aminoterminal zu dem Serin der Sequenz SxAQxR(LM)(WY)xL (Sequenz C1 der C-Domäne) entsprechen.

22. Verfahren zur maßgeschneiderten Synthese artifizieller nicht-ribosomaler Peptidsynthetase-Polyketidsynthase-Hybridsysteme (NRPS-PKS) nach Anspruch 17, **dadurch gekennzeichnet, daß gekennzeichnet**, daß die Fusion der einzelnen Domänen, Abfolgen von Domänen, Module oder Abfolgen von Modulen in DNA-Abschnitten erfolgt, die definierten Linkerbereichen der NRPS-PKS im Linkerbereich zwischen einer T- und einer darauffolgenden KS-Domäne zwischen den Aminosäuren 38 und 39 (jeweils einschließlich) carboxyterminal des Serins der Sequenz DxFFxxLGG(DH)S(IL) der T-Domäne entsprechen.

23. Verfahren zur maßgeschneiderten Synthese artifizieller nicht-ribosomaler Peptidsynthetase-Polyketidsynthase-Hybridsysteme (NRPS-PKS) nach Anspruch 17, **dadurch gekennzeichnet, daß** die Fusion der einzelnen Domänen, Abfolgen von Domänen, Module oder Abfolgen von Modulen in DNA-Abschnitten erfolgt, die definierten Linkerbereichen der NRPS-PKS im Linkerbereich zwischen einer ACP-Domäne und einer darauf folgenden Domäne zwischen den Aminosäuren 38 und 39 (jeweils einschließlich) carboxyterminal des 4'-Phosphopantethein-bindenden Serins der Sequenz (LI)GxDSL der ACP-Domäne entsprechen.

24. Verfahren zur maßgeschneiderten Synthese artifizieller nicht-ribosomaler Peptidsynthetase-Polyketidsynthase-Hybridsysteme (NRPS-PKS) nach Anspruch 17, **dadurch gekennzeichnet, daß** die Fusion der einzelnen Domänen, Abfolgen von Domänen, Module oder Abfolgen von Modulen in DNA-Abschnitten erfolgt, die definierten Linkerbereichen der NRPS im Linkerbereich zwischen einer A- und einer ACP-Domäne zwischen den Aminosäuren 16 und 17 (jeweils einschließlich) carboxyterminal zu dem Lysin der Sequenz NGK(VL)DR der A-Domäne und zwischen den Aminosäuren 46-77 aminoterminal zu dem 4'-Phosphopantethein-bindenden Serin der Sequenz (LI)GxDSL der ACP-Domäne entsprechen.

25. Verfahren gemäß Anspruch 16, das folgende Schritte einschließt:
ix) mittels eines rekombinanten Vektors, der die gemäß Schritten i) bis v) erzeugte DNA enthält, wird ein geeigneter Mikroorganismus transformiert, und
x) die transformierten Mikroorganismen werden kultiviert um die artifizielle Peptidsynthetase zu synthetisieren.

26. Verfahren zur maßgeschneiderten Synthese artifizieller nicht-ribosomaler Peptidsynthetasen (NRPS) nach Anspruch 16, **dadurch gekennzeichnet, daß** a1) eine DNA-Sequenz ausgewählt wird, die für eine natürlich vorkommende Abfolge von Domänen oder Modulen kodiert, die eine vorbestimmte Abfolge von Aminosäuren eines vorbestimmten Peptids anlagern kann, daß a2) aus dieser DNA-Sequenz ein vorbestimmter Anteil mittels an sich bekannter Methoden entfernt wird, der für ein oder mehrere Domänen oder Module kodiert, die eine Aminosäure oder eine vorbestimmte Abfolge von Aminosäuren des Peptids anlagern kann, und daß a3) die verbliebenen DNA-Teilsequenzen zur gewünschten NRPS mittels an sich bekannter Methoden fusioniert werden.

27. Verfahren zur maßgeschneiderten Synthese artifizieller nicht-ribosomaler Peptidsynthetasen (NRPS) nach Anspruch 16, **dadurch gekennzeichnet, daß** b1) eine DNA-Sequenz ausgewählt wird, die für eine natürlich vorkommende Abfolge von Domänen oder Modulen kodiert, die eine vorbestimmte Abfolge von Aminosäuren eines vorbestimmten Peptids anlagern kann, daß b2) aus dieser DNA-Sequenz ein vorbestimmter Anteil mittels an sich bekannter Methoden entfernt wird, der für ein oder mehrere Domänen oder Module kodiert, die eine Aminosäure oder eine vorbestimmte Abfolge von Aminosäuren des Peptids anlagern kann, und daß b3) die verbliebenen DNA-Teilsequenzen zusammen mit einer DNA-Sequenz, die für eine Domäne oder eine Abfolge von Domänen, für ein Modul oder eine Abfolge von Modulen kodiert, die eine vorbestimmte Aminosäure oder eine vorbestimmte Abfolge von Aminosäuren eines Peptids anlagern kann, zur gewünschten NRPS mittels an sich bekannter Methoden fusioniert werden.

28. Verwendung der maßgeschneiderte artifiziellen nicht-ribosomalen Peptidsynthetase nach Anspruch 1 zur nicht-ribosomalen Synthese von Peptiden einer vorbestimmten Länge und Zusammensetzung und/oder zur nicht-ribosomalen Modifikation von Peptiden oder Aminosäuren.

29. Verwendung der maßgeschneiderten artifiziellen nicht-ribosomalen Peptidsynthetase-Polyketidsynthase-Hybridsysteme (NRPS-PKS) nach Anspruch 3 zur nicht-ribosomalen Synthese von Peptid-Hybridsystemen enthaltend Acetat- und/oder Propionat-Einheiten einer vorbestimmten Länge und Zusammensetzung.

## Claims

1. A tailor-made artificial non-ribosomal peptide synthetase (NRPS) for non-ribosomal synthesis of peptides of predetermined length and composition and/or for non-ribosomal modification of peptides or amino acids, which NRPS comprises a plurality of domains whose sequence determines the structure and/or modification of the peptide or the amino acid, with an adenylation domain (A domain) and a thiolation domain (T domain), where appropriate a condensation domain (C domain) or a cyclization domain (Cy domain), where appropriate a thioesterase domain (Te domain) or a reductase domain (R domain), and also, where appropriate, at least one modification domain constituting a module, each module being characteristic for a predetermined amino acid and each module having linker regions between at least one of said domains and/or between successive modules, **characterized in that** said NRPS is obtainable by specific fusion of domains or of sequences of domains and/or of modules or of sequences of modules in one or more predetermined linker regions and **in that** the fusion has taken place in one or more of the following linker regions:
a) in the linker region between the T and the subsequent domain between amino acids 34 to 45 (in each case inclusively), carboxy-terminally to the 4'-phosphopantethein-binding serine in the sequence DxFFxxLGG(DH)S(IL) of the T domain, and/or
b) in the linker region between an A and a T domain in the region of amino acids 10-18 (in each case inclusively), carboxy-terminally of the lysine in the sequence NGK(VL)DR (sequence A10 of the A domain), and/or
c) in the linker region between a C and an A domain in the region of amino acids 38-60 (in each case inclusively), amino-terminally of the leucine in the sequence L(TS)YxEL (sequence A1 of the A domain), and/or
d) in the linker region between an E and a C domain in the region of amino acids 9-28 (in each case inclusively), amino-terminally of the serine in the sequence SxAQxR(LM)(WY)xL (sequence C1 of the C domain), and the sequences being denoted according to Marahiel et al. (1997) Chem. Rev. 97: pp.2651-2673.

2. The tailor-made artificial non-ribosomal peptide synthetase as claimed in claim 1, **characterized in that** the specific fusion has taken place in a linker region between a T domain and the subsequent domain, in a linker region between an A and a T domain, in a linker region between a C and an A domain and/or in a linker region between and E and a C domain.

3. A tailor-made artificial non-ribosomal peptide synthetase-polyketide synthase hybrid system (NRPS-PKS) as claimed in claim 1, **characterized in that** said system contains domains, sequences of domains, modules or sequences of modules, which derive from polyketide synthases (PKSs), with an acyltransferase (AT), an acyl carrier protein (ACP), where appropriate a ketosynthase (KS), and where appropriate reductive domains, constitute a module, each module being characteristic for the coenzyme-A ester of malonic acid or an α-substituted malonic acid and each module having linker regions between at least one of the domains and/or between successive modules, and said NRPS-PKS is obtainable by specific fusion of domains or of sequences of domains and/or of modules or of sequences of modules in one or more predetermined linker regions, with the fusion having taken place in one or more of the following linker regions:
e) in the linker region between a T domain and a KS domain following it, between amino acids 34 to 45 (in each case inclusively) carboxy-terminally of the serine in the sequence DxFFxxLGG(DH)S(IL) of the T domain, and/or
f) in the linker region between an ACP domain and a domain following it, between amino acids 34 to 45 (in each case inclusively) carboxy-terminally of the 4'-phosphopantethein-binding serine in the sequence (LI)GxDSL of the ACP domain, and/or
g) in the linker region between an A and an ACP domain between amino acids 10-18 (in each case inclusively) carboxy-terminally of the lysine in the sequence NGK(VL)DR of the A domain, and between amino acids 46-77 amino-terminally of the 4'-phosphopantethein-based serine in the sequence (LI)GxDSL of the ACP domain.

4. The tailor-made artificial non-ribosomal peptide synthetase as claimed in claim 1, **characterized in that** the modification domain is selected from the group consisting of the epimerization domain (E domain), the N-methylation domain (M domain), the N-formulation domain (F domain) and the oxidation domain (Ox domain).

5. The tailor-made artificial non-ribosomal peptide synthetase as claimed in claim 1, **characterized in that** said synthetase has at least one module comprising an A, a T and C domains and/or at least one module comprising an A, a T and Cy domains.

6. The tailor-made artificial non-ribosomal peptide synthetase as claimed in claim 1, **characterized in that** said synthase has at least one linker region which has been obtained by fusion between the T domain and a subsequent C, E, Cy, R or Te domain.

7. The tailor-made artificial non-ribosomal peptide synthetase as claimed in claim 4, **characterized in that** said synthetase has at least one linker region which has been obtained by fusion between the T domain and a subsequent KS domain.

8. The tailor-made artificial non-ribosomal peptide synthetase as claimed in claim 1, **characterized in that** said synthetase has at least one linker region which has been obtained by fusion between the T domain and the subsequent domain between amino acids 38 and 39 (in each case inclusively), carboxy-terminally of the 4'-phosphopantethein-binding serine in the sequence DxFFxxLGG(DH)S(IL) of the T domain.

9. The tailor-made artificial non-ribosomal peptide synthetase as claimed in claim 1, **characterized in that** said synthetase has at least one linker region which has been obtained by fusion between an A and a T domain in the region of amino acids 16 and 17 (in each case inclusively), carboxy-terminally of the lysine in the sequence NGK(VL)DR (sequence A10 of the A domain).

10. The tailor-made artificial non-ribosomal peptide synthetase as claimed in claim 1, **characterized in that** said synthetase has at least one linker region which has been obtained by fusion between a C and an A domain in the region of amino acids 47 and 48 (in each case inclusively), amino-terminally of the leucine in the sequence L(TS)YxEL (sequence A1 of the A domain).

11. The tailor-made artificial non-ribosomal peptide synthetase as claimed in claim 1, **characterized in that** said synthetase has at least one linker region which has been obtained by fusion in the linker region between an E and a C domain in the region of amino acids 20 and 21 (in each case inclusively), amino-terminally of the serine in the sequence SxAQxR(LM)(WY)xL (sequence C1 of the C domain).

12. The tailor-made artificial non-ribosomal peptide synthetase as claimed in claim 1, **characterized in that** said synthetase constitutes a module for modification of a given amino acid, which contains the A and T domains with affinity for the amino acid to be converted, the desired modification domain and, where appropriate, a Te domain or an R domain.

13. The tailor-made artificial non-ribosomal peptide synthetase as claimed in claim 4, **characterized in that** said synthetase has at least one linker region which has been obtained by fusion in the linker region between a T domain and a subsequent KS domain between amino acids 38 and 39 (in each case inclusively), carboxy-terminally of the serine in the sequence DxFFxxLGG(DH)S(IL) of the T domain.

14. The tailor-made artificial non-ribosomal peptide synthetase as claimed in claim 3, **characterized in that** said synthetase has at least one linker region which has been obtained by fusion in the linker region between an ACP domain and a domain following it between amino acids 38 and 39 (in each case inclusively), carboxy-terminally of the 4'-phosphopantethein-binding serine in the sequence (LI)GxDSL of the ACP domain.

15. The tailor-made artificial non-ribosomal peptide synthetase as claimed in claim 3, **characterized in that** said synthetase has at least one linker region which has been obtained by fusion in the linker region between an A domain and an ACP domain between amino acids 16 and 17 (in each case inclusively), carboxy-terminally of the lysine in the sequence NGK(VL)DR of the A domain, and between amino acids 46-77, amino-terminally of the 4'-phosphopantethein-binding serine in the sequence (LI)GxDSL of the ACP domain.

16. A method for tailor-made synthesis of artificial non-ribosomal peptide synthetases (NRPSs) as claimed in claim 1, comprising the following measures:
i) selecting a first DNA sequence which codes for a domain, a sequence of domains, a module or a sequence of modules, which can attach and convert a predetermined amino acid or a predetermined sequence of amino acids of the desired peptide,
ii) selecting a second DNA sequence which codes for a domain, a sequence of domains, a module or a sequence of modules, which can attach and convert another predetermined amino acid or another predetermined sequence of amino acids of the desired peptide,
iii) linking the 3' end of the first DNA sequence to the 5' and of the second DNA sequence,
iv) where appropriate, repeating steps ii) and iii) with DNA sequences of the desired length and composition, until attaining the length and composition according to the desired peptide, and
v) expressing the DNA obtained in this way according to methods known per se to give the corresponding polypeptide,
**characterized in that** the individual domains, sequences of domains, modules or sequences of modules are fused in DNA sections which correspond to the defined NRPS linker regions, with the defined NRPS linker regions being selected from the group consisting of
a) the linker region between the T and the subsequent domain between amino acids 34 and 45 (in each case inclusively), carboxy-terminally of the 4'-phosphopantethein-binding serine in the sequence DxFFxxLGG(DH)S(IL) of the T domain, and/or
b) the linker region between an A and a T domain in the region of amino acids 10-18 (in each case inclusive of), carboxy-terminally of the lysine in the sequence NGK(VL)DR (sequence A10 of the A domain), and/or
c) the linker region between a C and an A domain in the region of amino acids 38-60 (in each case inclusively), amino-terminally of the leucine in the sequence L(TS)YxEL (sequence A1 of the A domain), and/or
d) the linker region between an E and a C domain in the region of amino acids 9-28 (in each case inclusively), amino-terminally of the serine in the sequence SxAQxR(LM)(WY)xL (sequence C1 of the C domain), and the sequences being denoted according to Marahiel et al. (1997) Chem. Rev. 97: pp.2651-2673.

17. The method for tailor-made synthesis of artificial non-ribosomal peptide synthetase-polyketide synthase hybrid systems (NRPS-PKS) as claimed in claim 16, comprising the additional measures:
vi) selecting a DNA sequence which codes for a domain, a sequence of domains, a module or a sequence of modules, which can attach a predetermined acetate or propionate unit or a predetermined sequence of acetate and/or propionate units to the desired product,
vii) linking the 3' end of a DNA sequence from steps i), ii) or from repeating said steps to the 5' end of the DNA sequence from step vi), and
viii) where appropriate, repeating steps vi), i) and/or ii) with DNA sequences of the desired length and composition, until attaining the length and composition according to the desired peptide hybrid system containing acetate and/or propionate units, **characterized in that** the individual domains, sequences of domains, modules or sequences of modules are fused in DNA sections which correspond to the defined NRPS-PKS linker regions, with the defined NRPS-PKS linker regions being selected from the group consisting of
e) the linker region between a T domain and a KS domain following it, between amino acids 34 to 45 (in each case inclusively) carboxy-terminally of the serine in the sequence DxFFxxLGG(DH)S(IL) of the T domain, and/or
f) the linker region between an ACP domain and a domain following it, between amino acids 34 to 45 (in each case inclusively) carboxy-terminally of the 4'-phosphopantethein-binding serine in the sequence (LI)GxDSL of the ACP domain, and/or
g) the linker region between an A and an ACP domain between amino acids 10-18 (in each case inclusively) carboxy-terminally of the lysine in the sequence NGK(VL)DR of the A domain, and between amino acids 46-77 amino-terminally of the 4'phosphopantethein-binding serine in the sequence (LI)GxDSL of the ACP domain.

18. The method for tailor-made synthesis of artificial non-ribosomal peptide synthetases (NRPSs) as claimed in claim 16, **characterized in that characterized** that the individual domains, sequences of domains, modules or sequences of modules are fused in DNA sections which correspond to defined NRPS linker regions in the linker region between the T domain and the subsequent domain between amino acids 38 and 39 (in each case inclusively), carboxy-terminally of the 4'-phosphopantethein-binding serine in the sequence DxFFxxLGG(DH)S(IL) of the T domain.

19. The method for tailor-made synthesis of artificial non-ribosomal peptide synthetases (NRPSs) as claimed in claim 16, **characterized in that** the individual domains, sequences of domains, modules or sequences of modules are fused in DNA sections which correspond to defined NRPS linker regions in the linker region between an A domain and a T domain in the region of amino acids 16 and 17 (in each case inclusively), carboxy-terminally of the lysine in the sequence NGK(VL)DR (sequence A10 of the A domain).

20. The method for tailor-made synthesis of artificial non-ribosomal peptide synthetases (NRPSs) as claimed in claim 16, **characterized in that** the individual domains, sequences of domains, modules or sequences of modules are fused in DNA sections which correspond to defined NRPS linker regions in the linker region between a C domain and an A domain in the region of amino acids 47 and 48 (in each case inclusively), amino-terminally of the leucine in the sequence L(TS)YxEL (sequence A1 of the A domain).

21. The method for tailor-made synthesis of artificial non-ribosomal peptide synthetases (NRPSs) as claimed in claim 16, **characterized in that** the individual domains, sequences of domains, modules or sequences of modules are fused in DNA sections which correspond to defined NRPS linker regions in the linker region between an E domain and a C domain in the region of amino acids 20 and 21 (in each case inclusively), amino-terminally of the serine in the sequence SxAQxR(LM)(WY)xL (sequence C1 of the C domain).

22. The method for tailor-made synthesis of artificial non-ribosomal peptide synthetase-polyketide synthase hybrid systems (NRPS-PKS) as claimed in claim 17, **characterized in that characterized** that the individual domains, sequences of domains, modules or sequences of modules are fused in DNA sections which correspond to defined NRPS-PKS linker regions in the linker region between a T domain and a KS domain following it between amino acids 38 and 39 (in each case inclusively), carboxy-terminally of the serine in the sequence DXFFxxLGG(DH)S(IL) of the T domain.

23. The method for tailor-made synthesis of artificial non-ribosomal peptide synthetase-polyketide synthase hybrid systems (NRPS-PKS) as claimed in claim 17, **characterized in that** the individual domains, sequences of domains, modules or sequences of modules are fused in DNA sections which correspond to defined NRPS-PKS linker regions in the linker region between an ACP domain and a domain following it between amino acids 38 and 39 (in each case inclusively), carboxy-terminally of the 4'-phosphopantethein-binding serine in the sequence (LI)GxDSL of the ACP domain.

24. The method for tailor-made synthesis of artificial non-ribosomal peptide synthetase-polyketide synthase hybrid systems (NRPS-PKS) as claimed in claim 17, **characterized in that** the individual domains, sequences of domains, modules or sequences of modules are fused in DNA sections which correspond to defined NRPS linker regions in the linker region between an A domain and an ACP domain between amino acids 16 and 17 (in each case inclusively), carboxy-terminally of the lysine in the sequence NGK(VL)DR of the A domain, and between amino acids 46-77, amino-terminally of the 4'-phosphopantethein-binding serine in the sequence (LI)GxDSL of the ACP domain.

25. The method as claimed in claim 16, which includes the following steps:
ix) transforming a suitable microorganism by means of a recombinant vector which contains the DNA generated according to steps i) to v), and
x) culturing the transformed microorganisms in order to synthesize the artificial peptide synthetase.

26. The method for tailor-made synthesis of artificial non-ribosomal peptide synthetases (NRPSs) as claimed in claim 16, **characterized in that** a1) a DNA sequence is selected, which codes for a naturally occurring sequence of domains or modules, which can attach a predetermined sequence of amino acids of a predetermined peptide, **in that** a2) a predetermined portion which codes for one or more domains or modules which can attach an amino acid or a predetermined sequence of amino acids of the peptide, is removed from said DNA sequence by means of methods known per se, and **in that** a3) the remaining DNA part sequences are fused by means of methods known per se to give the desired NRPS.

27. The method for tailor-made synthesis of artificial non-ribosomal peptide synthetases (NRPSs) as claimed in claim 16, **characterized in that** b1) a DNA sequence is selected, which codes for a naturally occurring sequence of domains or modules, which can attach a predetermined sequence of amino acids of a predetermined peptide, **in that** b2) a predetermined portion which codes for one or more domains or modules, which can attach an amino acid or a predetermined sequence of amino acids of the peptide, is removed from said DNA sequence by means of methods known per se, and **in that** b3) the remaining DNA part sequences are fused together with a DNA sequence which codes for a domain or a sequence of domains, for a module or a sequence of modules, which can attach a predetermined amino acid or a predetermined sequence of amino acids of a peptide, by means of methods known per se to give the desired NRPS.

28. The use of the tailor-made artificial non-ribosomal peptide synthetase as claimed in claim 1 for non-ribosomal synthesis of peptides of a predetermined length and composition and/or for non-ribosomal modification of peptides or amino acids.

29. The use of the tailor-made artificial non-ribosomal peptide synthetase-polyketide synthase hybrid systems (NRPS-PKS) as claimed in claim 3 for non-ribosomal synthesis of peptide hybrid systems comprising acetate and/or propionate units of a predetermined length and composition.

## Revendications

1. Peptide synthétase non-ribosomique (NRPS) artificielle sur mesure pour la synthèse non ribosomique de peptides d'une longueur et d'une composition prédéterminées et/ou pour la modification non-ribosomique de peptides ou d'acides aminés, la NRPS comprenant plusieurs domaines, dont l'enchaînement détermine la structure du peptide et/ou influence la modification du peptide ou de l'acide aminé, un domaine d'adénylation (domaine A) et un domaine de thiolation (domaine T), éventuellement un domaine de condensation (domaine C) ou un domaine de cyclisation (domaine Cy), éventuellement un domaine thioestérase (domaine Te) ou un domaine réductase (domaine R), ainsi qu'éventuellement au moins un domaine de modification représentent un module, chaque module étant caractéristique d'un acide aminé pré-déterminé, et chaque module présente entre au moins un des domaines et/ou entre les modules successifs des régions linkers, **caractérisée en ce que** la NRPS peut être obtenue par fusion dirigée de domaines ou d'enchaînements de domaines et/ou de modules ou d'enchaînements de modules dans une ou plusieurs régions linkers prédéterminées, et **en ce que** la fusion est effectuée dans une ou plusieurs régions linkers ;
a) dans la région linker entre le domaine T et le domaine suivant entre les acides aminés de 34 à 45 (compris à chaque fois) à l'extrémité carboxy jusqu'à la sérine de la séquence DxFFxxLGG(DH)S(IL) du domaine T liant la 4'-phosphopantethéine, et/ou
b) dans la région linker entre un domaine A et un domaine T dans la région des acides aminés 10-18 (compris à chaque fois) à l'extrémité carboxy jusqu'à la lysine de la séquence NGK(VL)DR (séquence A10 du domaine A), et/ou
c) dans la région linker entre un domaine C et un domaine A dans la région des acides aminés 38-60 (compris à chaque fois) à l'extrémité amino jusqu'à la leucine de la séquence L(TS)YXEL (séquence A1 du domaine A), et/ou
d) dans la région linker entre un domaine E et un domaine C dans la région des acides aminés 9-28 (compris à chaque fois) à l'extrémité amino jusqu'à la sérine de la séquence SxAQxR (LM) (WY) xL (séquence C1 du domaine C), les séquences sont désignées d'après Marahiel et al. (1997), Chem. Rev. 97: p.2651-2673.

2. Peptide synthétase non-ribosomique artificielle sur mesure selon la revendication 1, **caractérisée en ce que** la fusion dirigée est effectuée dans une région linker entre un domaine T et le domaine suivant, dans une région linker entre un domaine A et un domaine T, dans une région linker entre un domaine C et un domaine A et/ou dans une région linker entre un domaine E et un domaine C.

3. Système hybride artificiel de peptide-synthétase-polycétide-synthase non-ribosomique (NRPS-PKS) sur mesure selon la revendication 1, **caractérisé en ce qu'**il comprend des domaines, des enchaînements de domaines, des modules ou enchaînements de modules, qui dérivent de polycétidesynthétases (PKS), où une acyltransférase (AT), une acyl-carrier protein (ACP), éventuellement une cétosynthase (KS), et éventuellement des domaines réducteurs représentent un module, chaque module étant caractéristique du coenzyme-A-ester de l'acide malonique ou d'un acide malonique substitué en α, et chaque module présentant des régions linkers entre au moins un des domaines et/ou entre les modules successifs, et on peut obtenir la NRPS-PKS par fusion dirigée de domaines ou d'enchaînement de domaines et/ou de modules ou d'enchaînement de modules dans une ou plusieurs régions linkers prédéterminés, la fusion étant effectuée dans une ou plusieurs des régions linkers :
e) dans la région linker entre un domaine T et un domaine KS suivant entre les acides aminés 34 à 45 (compris à chaque fois) à l'extrémité carboxy jusqu'à la sérine de la séquence DxFFxxLGG(DH)S ( (L) du domaine T, et/ou
f) dans la région linker entre un domaine ACP et un domaine suivant entre les acides aminés 34 à 45 (compris à chaque fois) à l'extrémité carboxy jusqu'à la sérine de la séquence (LI)GxDSL du domaine ACP liant la 4'-phosphopantethéiné et/ou
g) dans la région linker entre un domaine A et un domaine ACP entre les acides aminés 10-18 (compris à chaque fois) à l'extrémité carboxy jusqu'à la lysine de la séquence NGK(VL)DR du domaine A et entre les acides aminés 46-77 à l'extrémité amino jusqu'à la sérine de la séquence (U)GxDSL du domaine ACP liant la 4'-phosphopantethéine.

4. Peptide synthétase non-ribosomique artificielle sur mesure selon la revendication 1, **caractérisée en ce que** le domaine de modification est choisi dans le groupe constitué par le domaine d'épimérisation (domaine E), le domaine de N-méthylation (domaine M), le domaine de formylation (domaine F) ou le domaine d'oxydation (domaine Ox).

5. Peptide synthétase non-ribosomique artificielle sur mesure selon la revendication 1, **caractérisée en ce qu'**elle présente au moins un module comprenant un domaine A, T et C et/ou au moins un module comprenant un domaine A, T et Cy.

6. Peptide synthétase non-ribosomique artificielle sur mesure selon la revendication 1, **caractérisée en ce qu'**elle présente au moins une région linker que l'on obtient par fusion entre le domaine T et un des domaines suivants C, E, Cy, R ou Te.

7. Peptide synthétase non-ribosomique artificielle sur mesure selon la revendication 3, **caractérisée en ce qu'**elle présente au moins une région linker que l'on obtient par fusion entre le domaine T et un domaine KS suivant.

8. Peptide synthétase non-ribosomique artificielle sur mesure selon la revendication 1, **caractérisée en ce qu'**elle présente au moins une région linker que l'on obtient par fusion entre le domaine T et le domaine suivant entre les acides aminés 38 et 39 (compris à chaque fois) à l'extrémité carboxy jusqu'à la sérine de la séquence DxFFxxLGG(DH)S(IL) du domaine T liant la 4'-phosphopantethéine.

9. Peptide synthétase non-ribosomique artificielle sur mesure selon la revendication 1, **caractérisée en ce qu'**elle présente au moins une région linker que l'on obtient par fusion entre un domaine A et un domaine T dans la région des acides aminés 16 et 17 (compris à chaque fois) à l'extrémité carboxy jusqu'à la lysine de la séquence NGK (VL) DR (séquence A 10 du domaine A).

10. Peptide synthétase non-ribosomique artificielle sur mesure selon la revendication 1, **caractérisée en ce qu'**elle présente au moins une région linker que l'on obtient par fusion entre un domaine C et un domaine A dans la région des acides aminés 47 et 48 (compris à chaque fois) à l'extrémité amino jusqu'à la leucine de la séquence L(TS)YxEL (séquence A 1 du domaine A).

11. Peptide synthétase non-ribosomique artificielle sur mesure selon la revendication 1, **caractérisée en ce qu'**elle présente au moins une région linker que l'on obtient par fusion entre un domaine E et un domaine C dans la région des acides aminés 20 et 21 (compris à chaque fois) à l'extrémité amino jusqu'à la sérine de la séquence SxAQxR (LM) (WY) xL (séquence C1 du domaine C).

12. Peptide synthétase non-ribosomique artificielle sur mesure selon la revendication 1, **caractérisée en ce qu'**elle représente un module pour la modification d'un acide aminé prédéterminé, qui comprend les domaines A et T affins à l'acide aminé à transformer, le domaine de modification voulu et éventuellement un domaine Te ou un domaine R.

13. Peptide synthétase non-ribosomique artificielle sur mesure selon la revendication 3, **caractérisée en ce qu'**elle présente au moins une région linker que l'on obtient par fusion entre un domaine T et un domaine KS suivant entre les acides aminés 38 et 39 (compris à chaque fois) à l'extrémité carboxy jusqu'à la sérine de la séquence DxFFxxLGG(DH)S(tL) du domaine T.

14. Peptide synthétase non-ribosomique artificielle sur mesure selon la revendication 3, **caractérisée en ce qu'**elle présente au moins une région linker que l'on obtient par fusion entre un domaine ACP et un domaine suivant entre les acides aminés 38 et 39 (compris à chaque fois) à l'extrémité carboxy jusqu'à la sérine de la séquence (LI)GxDSL du domaine ACP liant la 4'-phosphopantethéine.

15. Peptide synthétase non-ribosomique artificielle sur mesure selon la revendication 3, **caractérisée en ce qu'**elle présente au moins une région linker, que l'on obtient par fusion dans la région linker entre un domaine A et un domaine ACP entre les acides aminés 16 et 17 (compris à chaque fois) à l'extrémité carboxy jusqu'à lysine de la séquence NGK(VL)DR du domaine A et entre les acides aminés 46-77 de l'extrémité amino jusqu'à la sérine de la séquence (LI)GxDSL du domaine ACP liant la 4'-phosphopantethéine.

16. Procédé pour la synthèse de peptide-synthétases non-ribosomiques artificielles (NRPS) sur mesure selon la revendication 1, comprenant les mesures :
i) choix d'une première séquence d'ADN codant un domaine, un enchaînement de domaines, un module ou un enchaînement de modules, qui peut fixer par addition et transformer un acide aminé pré-déterminé ou un enchaînement d'acides aminés prédéterminé du peptide voulu.
ii) choix d'une seconde séquence d'ADN, qui code un domaine, un enchaînement de domaines, un module ou un enchaînement de modules, qui peut fixer par addition et transformer un autre acide aminé prédéterminé ou un autre enchaînement. d'acides aminés prédéterminé du peptide voulu.
iii) composé de l'extrémité 3' de la première séquence d'ADN avec l'extrémité 5' de la seconde séquence d'ADN,
iv) éventuellement répétition des étapes ii) et iii) avec les séquences d'ADN de longueur et de composition voulues, jusqu'à ce que la longueur et la composition correspondantes au peptide voulu soient atteintes, et
v) expression de l'ADN ainsi obtenu dans le polypeptide correspondant selon des procédés connus en soi,
**caractérisé en ce que** la fusion des différents domaines, des enchaînements de domaines, des modules ou des enchaînements de modules est effectuée dans des segments d'ADN, qui correspondent à des régions linkers définies de la NRPS, les régions linkers définies de la NRPS sont prises dans le groupe constitué par :
a) la région linker entre le domaine T et le domaine suivant entre les acides aminés 34 à 45 (compris à chaque fois) à l'extrémité carboxy jusqu'à la sérine de la séquence DxFFxxLGG(DH)S(IL) du domaine T liant la 4'-phosphopantethéine, et/ou
b) la région linker entre un domaine A et un domaine T dans la région des acides aminés 10-18 (compris à chaque fois) à l'extrémité carboxy jusqu'à la lysine de la séquence NGK(VL)DR (séquence A10 du domaine A), et/ou
c) la région linker entre un domaine C et un domaine A dans le domaine des acides aminés 38-60 (compris à chaque fois) de l'extrémité amino jusqu'à la leucine de la séquence L(TS)YxEL (séquence A1 du domaine A), et/ou
d) la région linker entre un domaine E et un domaine C dans la région des acides aminés 9-28 (compris à chaque fois) de l'extrémité amino jusqu'à la sérine de la séquence SxAQxR(LM)(WY)xL (séquence C1 du domaine C), les séquences désignées selon Marahiel et al. (1997), Chem. Rev. 97: p.2651-2673.

17. Procédé pour la synthèse de systèmes hybrides artificiels de peptide-synthétase-polycétide-synthase non-ribosomiques (NRPS-PKS) sur mesure selon la revendication 16, comprenant les mesures supplémentaires :
vi) choix d'une séquence d'ADN, qui code un domaine, un enchaînement de domaines, un module ou enchaînement de modules, qui peut fixer par addition une unité prédéterminée acétate ou propionate ou un enchaînement prédéterminé d'unités acétate et/ou propionate dans le produit voulu,
vii) liaison de l'extrémité 3' d'une séquence d'ADN des étapes i), ii) ou de la répétition de ces étapes avec l'extrémité 5' de la séquence d'ADN de l'étape vi), et
viii) éventuellement répétition des étapes vi), i) et/ou ii) avec des séquences d'ADN d'une longueur et d'une composition voulues, jusqu'à ce qu'on atteigne la longueur et la composition correspondantes au système hybride peptidique voulu renfermant des unités acétate et/ou propionate, **caractérisé en ce que** la fusion des différents domaines, des enchaînements de domaines, des modules ou des enchaînements de modules est effectuée dans des segments d'ADN, qui correspondent à des régions linkers définies de la NRPS-PKS, les régions linkers définies de la NRPS-PKS sont prises dans le groupe constitué par
e) la région linker entre un domaine T et un domaine KS suivant entre les acides aminés 34 à 45 (compris à chaque fois) à l'extrémité carboxy jusqu'à la sérine de la séquence DxFFxxLGG(DH)S{{L) du domaine T, et/ou
f) la région linker entre un domaine ACP et un domaine suivant entre les acides aminés 34 à 45 (compris à chaque fois) à l'extrémité carboxy jusqu'à la sérine de la séquence (LI)GxDSL du domaine ACP liant la 4'-phosphopantethéine, et/ou
g) la région linker entre un domaine A et un domaine ACP entre les acides aminés 10-18 (compris à chaque fois) à l'extrémité carboxy jusqu'à la lysine de la séquence NGK(VL)DR du domaine A et entre les acides aminés 46-77 de l'extrémité amino jusqu'à la sérine de la séquence (LI)GXDSL du domaine ACP liant la 4'-phosphopantethéine.

18. Procédé pour la synthèse de peptide-synthétases non-ribosomiques (NRPS) artificielles sur mesure selon la revendication 16, **caractérisé en ce que** la fusion des différents domaines, des enchaînements de domaines, des modules ou des enchaînements de modules est effectuée dans des segments d'ADN, qui correspondent à des régions linkers définies de la NRPS dans la région linker entre le domaine T et le domaine suivant entre les acides aminés 38 et 39 (compris à chaque fois) à l'extrémité carboxy jusqu'à la sérine de la séquence DxFFxxLGG(DH)S(IL) du domaine T liant la 4'-phosphopantethéine.

19. Procédé pour la synthèse de peptide-synthétases non-ribosomiques (NRPS) artificielles sur mesure selon la revendication 16, **caractérisé en ce que** la fusion des différents domaines, des enchaînements de domaines, des modules ou des enchaînements de modules est effectuée dans des segments d'ADN, qui correspondent à des régions linkers définies de la NRPS dans la région linker entre un domaine A et un domaine T dans la région des acides aminés 16 et 17 (compris à chaque fois) à l'extrémité carboxy jusqu'à la lysine de la séquence NGK(VL)DR (séquence A10 du domaine A).

20. Procédé pour la synthèse de peptide-synthétases non-ribosomiques (NRPS) artificielles sur mesure selon la revendication 16, **caractérisé en ce que** la fusion des différents domaines, des enchaînements de domaines, des modules ou des enchaînements de modules est effectuée dans des segments d'ADN, qui correspondent à des régions linker définies de la NRPS dans la région linker entre un domaine C et un domaine A dans la région des acides aminés 47 et 48 (compris à chaque fois) à l'extrémité amino jusqu'à la leucine de la séquence L(TS)YxEL (séquence A1 du domaine A).

21. Procédé pour la synthèse de peptide-synthétases non-ribosomiques (NRPS) artificielles sur mesure selon la revendication 16, **caractérisé en ce que** la fusion des différents domaines, des enchaînements de domaines, des modules ou des enchaînements de modules est effectuée dans des segments d'ADN, qui correspondent à des régions linkers définies de la NRPS dans la région linker entre un domaine E et un domaine D dans la région des acides aminés 20 et 21 (compris à chaque fois) à l'extrémité amino jusqu'à la sérine de la séquence SxAQxR(LM)(WY)xL (séquence C1 du domaine C).

22. Procédé pour la synthèse de systèmes hybrides artificiels de peptide-synthétase-polycétide-synthase non-ribosomiques (NRPS-PKS) sur mesure selon la revendication 17, **caractérisé en ce que** la fusion des différents domaines, des enchaînements de domaines, des modules ou des enchaînements de modules est effectuée dans des segments d'ADN, qui correspondent à des régions linkers définies de la NRPS-PKS dans la région linker entre un domaine T et un domaine KS suivant entre les acides aminés 38 et 39 (compris à chaque fois) à l'extrémité jusqu'à la sérine de la séquence DxFFxxLGG(DH)S(IL) du domaine T.

23. Procédé pour la synthèse de systèmes hybrides artificiels de peptide-synthétase-polycétide-synthase non-ribosomiques (NRPS-PKS) sur mesure selon la revendication 17, **caractérisé en ce que** la fusion des différents domaines, des enchaînements de domaines, des modules ou des enchaînements de modules est effectuée dans des segments d'ADN, qui correspondent à des régions linkers définies de la NRPS-PKS dans la région linker entre un domaine ACP et un domaine suivant entre les acides aminés 38 et 39 (compris à chaque fois) à l'extrémité carboxy jusqu'à la sérine de la séquence (LI)GxDSL du domaine ACP liant la 4'-phosphopantethéine.

24. Procédé pour la synthèse de systèmes hybrides artificiels de peptide-synthétase-polycétide-synthase non-ribosomiques (NRPS-PKS) sur mesure selon la revendication 17, **caractérisé en ce que** la fusion des différents domaines, des enchaînements de domaines, des modules ou des enchaînements de modules est effectuée dans des segments d'ADN, qui correspondent à des régions linkers définies de la NRPS dans la région linker entre un domaine A et un domaine ACP entre les acides aminés 16 et 17 (compris à chaque fois) à l'extrémité carboxy jusqu'à la lysine de la séquence NGK(VL)DR du domaine A et entre les acides aminés 46-77 à l'extrémité amino jusqu'à la sérine de la séquence (LI)GxDSL du domaine ACP liant la 4'-phosphopantethéine.

25. Procédé selon la revendication 16, comprenant les étapes suivantes :
ix) on transforme un microorganisme approprié à l'aide d'un vecteur recombinant qui renferme l'ADN produit selon les étapes i) à v), et
x) on cultive les microorganismes transformés afin de synthétiser la peptide-synthétase artificielle.

26. Procédé pour la synthèse de peptide-synthétases non-ribosomiques (NRPS) artificielles sur mesure selon la revendication 16, **caractérisé en ce que** a1) on choisit une séquence d'ADN, qui code un enchaînement de domaines ou de modules existants dans la nature, qui peut fixer par addition un enchaînement prédéterminé d'acides aminés d'un peptide prédéterminé, **en ce que** a2) on élimine à partir de cette séquence d'ADN à l'aide de procédés connus un fragment prédéterminé, qui code un ou plusieurs domaines ou modules, qui peuvent fixer par addition un acide aminé ou un enchaînement prédéterminé d'acides aminés du peptide, et **en ce que** a3) on fusionne les séquences partielles d'ADN restantes en vue d'obtenir la NRPS voulue à l'aide de méthodes connues en soi.

27. Procédé pour la synthèse de peptide-synthétases non-ribosomiques (NRPS) artificielles sur mesure selon la revendication 16, **caractérisé en ce que** b1) on choisit une séquence d'ADN, qui code un enchaînement de domaines ou de modules existant dans la nature, qui peut fixer par addition un enchaînement prédéterminé d'acides aminés d'un peptide prédéterminé, **en ce que** b2) on élimine à partir de cette séquence d'ADN, à l'aide de procédés connus, un fragment prédéterminé qui code un ou plusieurs domaines ou modules, qui peut fixer par addition un acide aminé ou un enchaînement prédéterminé en acides aminés du peptide, et **en ce que** b3) on fusionne à l'aide de méthodes connues en soi les séquences partielles d'ADN restantes conjointement avec une séquence d'ADN qui codé un domaine ou un enchaînement de domaines, un module ou un enchaînement de modules, qui peut fixer par addition un acide aminé prédéterminé ou un enchaînement d'acides aminés prédéterminé d'un peptide, en vue d'obtenir la NRPS voulue.

28. Utilisation de la peptide-synthétase non-ribosomique artificielle selon la revendication 1 pour la synthèse non-ribosomique de peptides d'une longueur et d'une composition prédéterminées et/ou pour la modification non-ribosomique de peptides ou d'acides aminés.

29. Utilisation de systèmes hybrides artificiels de peptide-synthétase-polycétidesynthase non-ribosomiques (NRPS-PKS) sur mesure selon la revendication 3 pour la synthèse non-ribosomique de systèmes hybrides de peptides renfermant des unités acétate et/ou propionate d'une longueur et d'une composition prédéterminées.
